# EUROPEAN PATENT APPLICATION

(11) **EP 1 911 481 A2**
(43) Date of publication of application: **16.04.2008**
(21) Application number: 07018536.8
(22) Date of filing: 20.09.2007
(51) Int. Cl.: A61M 15/00, A61M 16/06

(54) **Methods and systems of delivering medication via inhalation**

(30) Priority: 20.09.2006 US 826271 P; 25.10.2006 US 552871; 26.01.2007 US 627692
(71) Applicant: Next Safety, Inc., West Jefferson, NC 28690 (US)
(72) Inventor: Lemahieu, Edward, San Jose, CA 95125 (US); Jones, Charles, Jefferson, NC 28640 (US); Stern, Tom, Charlotte, NC 28214 (US); Hebrank, Jack, Durham, NC 27712 (US); Hunter, Charles Eric, Jefferson, NC 28640 (US); Duvall, Lyndell, Fleetwood, NC 28626 (US); Hartley, Chris, Boone, NC 28607 (US); Ballou, Jr. Bernard L., Raleigh, NC 27614 (US); Hunter, Jocelyn, Jefferson, NC 28640 (US); McNeil, Laurie Dept. of Physics and Astronomy, Chapel Hill, NC 27599-3255 (US); Wetzel, Paul, Jefferson, NC 28640 (US); Criss, Ron, West Jefferson, NC 28694 (US)
(74) Representative: Rehberg Hüppe + Partner

(57) **Abstract**

Systems and methods for delivery of a drug to the respiratory system of a patient where the drug is supplied at a positive pressure relative to atmospheric pressure are provided. In particular, the drugs are delivered to the respiratory system of a patient who is capable of unassisted breathing. With the systems and methods of the present disclosure, medication available in a variety of forms is introduced in a controlled fashion into the air stream in aerosol, nebulized, or vaporized form.

## Description

### RELATED APPLICATIONS

This application is a continuation-in-part of application Serial No. 11/552,871 filed October 25, 2006 that claims priority to and the benefit of provisional application, Serial No. 60/826,271 filed September 20, 2006.

### FIELD OF THE INVENTION

The present disclosure relates to the delivery of medications by inhalation. Specifically, it relates to the delivery of medications using purified air at a positive pressure with delivery coordinated in time with the respiratory cycle of the user.

### BACKGROUND

Earlier applications of the present applicant have recognized the dire consequences that polluted air, whether polluted by chemical agents or biological pathogens, has on our health, and has proposed a new family of clean air systems. In particular, since the Industrial Revolution, the respiratory systems of human beings have been continuously exposed to heightened levels of airborne pollutants. For people who live in urban or suburban areas today, there is no escape from airborne contaminants such as particulate exhaust, ozone, dust, mold, and the many other pollutants in outdoor city air. Studies show that in the housing of even the most affluent city dwellers, indoor air can be, and often is, dirtier than the air outside. As a practical matter, people who live in cities, whether in developed or developing nations, and regardless of their affluence, have been and continue to be without any defense against the effects of dirty air. In rural areas in much of the world air pollution conditions are as problematic as those found in cities, due in part to the location of fossil fuel power plants and, in developing nations, the widespread presence of factories and motor vehicles without any effective pollution controls.

In fact studies show that there are not only direct, immediate effects from breathing contaminated air, *e.g.*, as caused by exposure to air borne pathogens or toxic gases, but also long term consequences. The human respiratory system has not had time to develop a defense against today's air contamination and, as a result, public health suffers in the form of various pulmonary diseases, including an alarming increase in the incidence of asthma and pulmonary fibrosis as well as other diseases such as cancer, colds, and flu caused by breathing in pollutants.

In addition to the short and long term consequences, it will be appreciated that while some pollutants affect only the people directly exposed to the polluted air, other pollutants such as certain pathogens cause disease that can spread to others, with the potential of escalating into pandemics.

In response to these dangers, the present applicant has developed a family of portable breathing devices for providing the user with clean air. However, in addition to removing harmful substances, much benefit can be realized by then adding beneficial substances (*e.g.*, medicines) to the same air.

The architecture of the lung is designed to facilitate gas exchange, specifically oxygen and carbon dioxide, which are required to sustain life. The surface area of the adult human lung ranges between 50 and 100 square meters (538 and 1076 square feet). This surface area is comparable to the square footage of a small apartment. The surface area of the lung is 25 to 50 times greater than the surface area of the skin on an average size adult male. This extensive surface area in the lung makes it a preferred target for systemic delivery of drugs. Humans are well aware of the ability of the lung to absorb drugs. 400 billion cigarettes were sold in the United States in 2001 alone. These sales were driven by the desire for the systemic absorption of nicotine. Nicotine is not the only drug readily absorbed from the lung. Other drugs of abuse are preferentially inhaled because they are readily absorbed into the bloodstream and quickly transported to the brain without having to contend with the metabolizing effects of the liver that orally ingested medicines are subject to.

Historically, the inhaled route of medication delivery has been used to treat diseases of the lung. It is also the preferred route for non-invasive drug delivery for systemic delivery of medications. This would allow treatment of a variety of diseases that are affecting organ systems other than the lung. The benefits of the inhaled route include rapid absorption, avoidance of metabolism by the liver, and the absence of discomfort and complications associated with the intravenous or intramuscular route.

The inhaled route for systemic delivery of medications has not been fully utilized to date because of the absence of a practical delivery device. The most popular methods of delivering inhaled medications include nebulizers, pressurized multi dose inhalers, and dry powder inhalers. Each device is accompanied by multiple issues that complicate its use. In addition, the devices share technical impediments that complicate clinical use. The impediments that are common to all current methods of drug delivery are difficulty of coordination with patient respiratory pattern, interaction of the delivered medication with pollutants including ozone, and the reliance on the patient to supply the energy needed to inhale the medication (which is difficult for those with compromised respiratory systems).

Nebulizers use pressurized gas to create respirable droplet aerosols less than 5 micrometers in diameter. Ultrasound nebulizers have also been developed but could not be used because of their inability to nebulize suspension formulations. Issues that complicate the use of pressurized gas nebulizers include the need for a compressed gas supply that significantly limits portability, the need for frequent cleaning of the device to prevent bacterial colonization, the flooding of the market with poorly designed, cheaply manufactured nebulizers and the variability of the delivered dose (usually only 20-25% of the instilled dose in high cost systems).

Pressurized multi-dose inhalers are historically the most common delivery system for inhaled medications. Chlorofluorocarbons were initially used as a vehicle for these devices but these have subsequently been replaced due to environmental concerns. This bolus method of delivery causes a wide variation in the amount of medicine delivered to patients. The bolus of medication will deposit in different levels of the pulmonary tree depending on the timing of the delivery of the bolus in relation to the inhalation cycle. Therefore, the dose depositing in the airways in vivo is different than that measured in the laboratory setting. Education and compliance are major issues. Proportions of the "metered dose" are lost in the mouthpiece and oropharynx. Spacers and reservoirs have been developed to try to improve on this technology, however a highly coordinated effort is still needed.

Dry powder inhalers try to improve this need for a coordinated delivery effort by making the systems passive. In other words the patient provides the power required to deliver the medicine to the lung. There are several dry powder inhalers on the market all with proprietary techniques and design. This in itself causes complications in that a patient may have to learn several different techniques if they are taking multiple medications. In addition, small volume powder metering is not as precise as the measurement of liquids. Finally the ambient environmental conditions, especially humidity, can effect the dose of the drug reaching the lungs. A mistake as simple as exhaling into the device can effect drug delivery.

Obviously, by removing harmful contaminants from the air, providing it to the user at positive pressure, and then adding beneficial substances in precisely controlled concentrations and at the correct moments during the respiratory cycle for optimum benefit and efficiency, the optimal conditions for improving the health of countless individuals worldwide is realized. The present application seeks to address the above issues.

### SUMMARY

Disclosed are methods and systems for delivery of pharmaceutical compositions in high purity air at a positive pressure relative to atmospheric pressure. In some exemplary embodiments, methods and systems for delivery of pharmaceutical compositions in high purity, ozone-free air are provided.

One method of administering a pharmaceutical composition includes the following steps: providing the pharmaceutical composition in a gaseous, vaporized, nebulized, or aerosol form; introducing the pharmaceutical composition into a purified air stream of air filtered to a particle size of no greater than about 10-20 nanometers; and administering the pharmaceutical composition to a host in need of treatment via inhalation of the pharmaceutical composition in the purified air stream. In one embodiment, a very small volume of the pharmaceutical composition(s) is delivered along with a very large volume of airflow, allowing excellent dosage control relative to metered dose inhalers (MDI).

In addition to combining precise dosage control and a highly purified air stream, systems of the present disclosure also provide a means for precisely controlling the temperature and humidity of the air delivered to the user. Additionally, systems of the present disclosure (*e.g.*, via control circuitry) will allow dosing to be synchronized with the user's respiratory cycle allowing, for instance, drug delivery to the user only during inhalation. The delivery is aided by the positive pressure generated in the system, thereby requiring minimum effort by the user. This is particularly important with patients at the extremes of age (young and old) and those who are mentally unsound or intellectually challenged.

One embodiment of a system for delivery of pharmaceutical compositions includes the following: a purified air stream generator for generating a filtered air stream at a positive pressure, a face mask connected via a hose or other conduit to the air source, and means for introducing medication in gaseous, vaporized, or nebulized form into the air stream.

In particular, embodiments of the present disclosure include methods of administering drugs to the respiratory system of a patient, where the drug is delivered using purified air supplied at a positive pressure relative to atmospheric pressure. Other embodimetns of the present disclosure include administering medicines to the respiratory system of a patient including delivering the drug to the patient using purified air supplied at a positive pressure relative to atmospheric pressure, where the drug is delivered to correspond in time with an inhalation portion of a respiratory cycle of the patient, and where information from one or more devices used to monitor a condition of the patient are used to adjust a rate and a timing of delivery of the drug to the patient.

Embodiments of the present disclosure also include methods and devices of administering drugs to the respiratory system of a patient by delivering the drug to the patient at a positive pressure relative to atmospheric pressure, where the patient is capable of unassisted breathing. In embodiments, the drug is supplied in air, purified air, or a mixture of gases that is supplied at a positive pressure relative to atmospheric pressure.

Other systems, methods, features, and advantages of the present disclosure will be or become apparent to one with skill in the art upon examination of the following drawings and detailed description. It is intended that all such additional systems, methods, features, and advantages be included within this description, be within the scope of the present disclosure, and be protected by the accompanying claims.

### BRIEF DESCRIPTION OF THE DRAWINGS

Many aspects of the present disclosure can be better understood with reference to the following drawings. The components in the drawings are not necessarily to scale, emphasis instead being placed upon clearly illustrating the principles of the present disclosure. Moreover, in the drawings, like reference numerals designate corresponding parts throughout the several views.
FIG. 1 shows a three dimensional view of a prior art albuterol-containing aerosol canister for treating asthma.
FIG. 2A shows a front view and FIG. 2B shows a side view of one embodiment of a system of the present disclosure.
FIG. 3 shows a front view of an embodiment of the disclosed device.
FIG. 4 shows a sectional side view of an embodiment of the disclosed medi port.
FIG. 5 shows a sectional side view of one embodiment of an adapter for use with the mixing chamber of the medi port of FIG. 4.
FIG. 6 shows a sectional side view of an embodiment of the disclosed mixing chamber.
FIG. 7 shows a sectional side view of an embodiment of an adapter for use with the mixing chamber of FIG. 6.
FIG. 8 shows a sectional side view of an embodiment of the disclosed medi port.
FIG. 9 show a sectional side view of an embodiment of the disclosed mixing chamber.
FIG. 10 shows a sectional side view of an embodiment of an adapter for use with the mixing chamber of FIG. 9.
FIG. 11 shows a sectional side view of an embodiment of a medi port connected to a hose.
FIGS. 12-14 show embodiments of medi ports of the present disclosure.
FIGS. 15 and 16 illustrate a sectional side view of embodiments of the disclosed medi port.
FIG. 17 illustrates side and front views of an embodiment of the disclosed medi port connected to an embodiment of the face mask of the present disclosure.
FIG. 18 illustrates side and front views of another embodiment of the disclosed medi port connected to an embodiment of the face mask of the present disclosure.
FIG. 19 illustrates an embodiment of the system of the present disclosure where the medical port is configured for networked data communications.
FIG. 20 shows an embodiment of the medical port that features multiple ampules for delivery of multiple drugs.
FIG. 21 shows an embodiment of the blower and medical port that utilizes an air reservoir or bladder.
FIG. 22 is a graph of filter efficiency versus face velocity for 100 nm particles for standard filter materials tested.

### DETAILED DESCRIPTION

Before the present disclosure is described in greater detail, it is to be understood that this disclosure is not limited to particular embodiments described, and as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present disclosure is not entitled to antedate such publication by virtue of prior disclosure. Further, the dates of publication provided could be different from the actual publication dates that may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope or spirit of the present disclosure. Any recited method can be carried out in the order of events recited or in any other order that is logically possible.

The following examples are put forth so as to provide those of ordinary skill in the art with a complete disclosure and description of how to perform the methods and use the compositions and compounds disclosed and claimed herein. Efforts have been made to ensure accuracy with respect to numbers (*e.g.*, amounts, temperature, *etc.*), but some errors and deviations should be accounted for. Unless indicated otherwise, parts are parts by weight, temperature is in °C, and pressure is at or near atmospheric. Standard temperature and pressure are defined as 20 °C and 1 atmosphere.

Embodiments of the present disclosure will employ, unless otherwise indicated, techniques of synthetic organic chemistry, biochemistry, pharmacology, medicine, and the like, which are within the skill of the art. Such techniques are explained fully in the literature.

It must be noted that, as used in the specification and the appended claims, the singular forms "a," "an," and "the" include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

Prior to describing the various embodiments, the following definitions are provided and should be used unless otherwise indicated.

### Definitions:

As used herein the term "aerosol" refers to a suspension of solid or liquid particles in a gas.

As used herein the term "genetic material" generally refers to material that includes a biologically active component, including but not limited to nucleic acids (*e.g.*, single or double stranded DNA or RNA or siRNA's), proteins, peptides, polypeptides, and the like.

As used herein the term "surfactant" or "pulmonary surfactant" generally refers to specific lipo-protein substances naturally produced in the lungs that are essential for proper breathing, alveolar stability and gas exchange. Pulmonary surfactants are surface-active agents naturally formed by type II alveolar cells that reduce the surface tension at the air-liquid interface of alveoli. Pulmonary surfactants are generally made up of about 90% lipids (about half of which is the phospolipid dipalmitoylphosphatidylcholine (DPPC)) and about 10% protein. At least four native surfactants have been identified: SP-A, B, C, and D. The hydrophobic surfactant proteins B (SP-B) and C (SP-C) are tightly bound to the phospholipids, and promote their adsorption into the air-liquid interface of the alveoli. These proteins are critical for formation of the surfactant film. The term "surfactant" also includes currently available surfactant preparations, including, but not limited to, Survanta® (beractant), Infasurf® (calfactant), Exosurf neonatal® (colfosceril palmitate), Curosurf® (poractant alfa), Surfaxin® (lucinactant), Aerosurf® (aerosolized Surfaxin®), Vanticute® (lusupultide), Alveofact® (bovactant), as well as preparations being developed.

As used herein, the term "purified air" refers to air that has been synthesized from pure gasses or environmental air that has been filtered to reduce the amount of particulate matter and/or other contaminants such as, but not limited to, ozone, SO₂, and NO₂. While such contaminants may not be entirely removed/eliminated, the amount may be reduced from the amount found in the air of a particular environment and preferably reduced from the amount in air filtered with the use of HEPA grade filters. In some preferred embodiments, purified air includes less than about .03 % of particulate matter having a particle size greater than about 20 nm, as compared to the amount of particulate matter in the environmental air being purified. In some preferred embodiments the purified air includes less than about 0001% of the particle count of the environmental air being purified. In embodiments, purified air includes a reduced amount of ozone, as compared to the environmental air being purified. In some embodiments, purified air includes a reduced amount of of SO₂, as compared to the environmental air being purified, and in some embodiments includes a reduced amount of NO₂ as compared to the environmental air being purified. In some preferred embodiments, the purified air has a reduced amount of ozone, a reduced amount of of SO₂, and/or a reduced amount of NO₂, and a particle count less than about .03% than the particle counts of the environmental air being purified.

As used herein, the term "positive pressure" refers to a pressure of the air being supplied to the patient being greater than the atmospheric pressure.

As used herein, the terms "user", "host", and/or "patient" include humans and other living species that are in need of treatment and capable of being ventilated or of using the disclosed respirator. In particular, the terms "user", "host" and/or "patient" includes humans and mammals (*e.g.*, cats, dogs, horses, chicken, pigs, hogs, cows, and other cattle).

As used herein the term "pharmaceutical drug" generally refers to any pharmaceutically effective compound used in the treatment of any disease or condition. For example, the pharmaceutical drug can be used in the treatment of diseases such as asthma, bronchitis, emphysema, lung infection, cystic fibrosis, AAT deficiency, COPD, ARDS, IRDS, BPD, and MAS, among many other conditions. Useful pharmaceutical drugs that can be delivered via inhalation according to the disclosed methods include, but are not limited to, those that are listed within the Physician's Desk Reference (most recent edition, *e.g.*, 2007), published by Thomson PDR. Such drugs include, but are not limited to those set forth hereinafter in Table 1, which drugs can be administered with the disclosed device for the correlated indication. Table 1 provides a list of exemplary drugs that can be delivered via the instantly-disclosed device, all of which have been approved by the U.S. Food and Drug Administration for pulmonary delivery. Other drugs may be used in the presently disclosed methods, and the following list is not intended to be exhaustive.

**Table 1**

| | |
|---|---|
| ALBUTEROL | For the relief and prevention of bronchospasm in patients with reversible obstructive airway disease; acute attacks of bronchospasm (inhalation solution);prevention of exercise-induced bronchospasm. |
| ALBUTEROL SULFATE | For the relief of bronchospasm in patients 2 years of age and older with reversible obstructive airway disease and acute attacks of bronchospasm. For the treatment or prevention of bronchospasm in adults and children 4 years of age and older with reversible obstructive airway disease and for the prevention of exercise-induced bronchospasm in patients 4 years of age and older. |
| ATROPINE SULFATE | For the treatment or prevention of bronchospasm in adults and children 4 years of age and older with reversible obstructive airway disease and for the prevention of exercise-induced bronchospasm in patients 4 years of age and older. |
| BITOLTEROL MESYLATE | For prophylaxis and treatment of bronchial asthma and reversible bronchospasm. May be used with concurrent theophylline or steroid therapy. |
| BUDESONIDE | For the maintenance treatment of asthma as prophylactic therapy in adult and pediatric patients 6 years of age or older |
| CROMOLYN SODIUM | As prophylactic management of bronchial asthma. Cromolyn is given on a regular, daily basis in patients with frequent symptomatology requiring a continuous medication regimen. To prevent acute bronchospasm induced by exercise, toluene diisocyanate, environmental pollutants, and known antigens. |
| DESFLURANE | For induction or maintenance of anesthesia for inpatient and outpatient surgery in adults. |
| DEXAMETHASONE SODIUM PHOSPHATE | Maintenance treatment of asthma as prophylactic therapy in patients 5 years of age and older. |
| DORNASE ALFA | Daily administration of dornase alfa in conjunction with standard therapies is indicated in the management of cystic fibrosis patients to improve pulmonary function. In patients with an FVC greater than or equal to 40% of predicted, daily administration of dornase alfa has also been shown to reduce the risk of respiratory tract infections requiring parenteral antibiotics. |
| ENFLURANE | For induction and maintenance of general anesthesia. Enflurane may be used to provide analgesia for vaginal delivery. Low concentrations of enflurane may also be used to supplement other general anesthetic agents during delivery by Cesarean section. Higher concentrations of enflurane may produce uterine relaxation and an increase in uterine bleeding. |
| | |
| EPINEPHRINE | For temporary relief of shortness of breath, tightness of chest, and wheezing due to bronchial asthma. |
| ERGOTAMINE TARTRATE | As therapy to abort or prevent vascular headache, (eg, migraine, migraine variants, or so called "histaminic cephalalgia"). |
| FLUNISOLIDE | For the maintenance treatment of asthma as prophylactic therapy in adult and pediatric patients 6 years of age and older. It is also indicated for asthma patients requiring oral corticosteroid therapy, where adding flunisolide HFA inhalation aerosol may reduce or eliminate the need for oral corticosteroids. |
| FLUTICASONE PROPIONATE | For the maintenance treatment of asthma as prophylactic therapy in patients 4 years of age and older. Also indicated for patients requiring oral corticosteroid therapy for asthma. |
| | |
| FORMOTEROL FUMARATE | For long-term, twice-daily (morning and evening) administration in the maintenance treatment of asthma and in the prevention of bronchospasm in adults and children 5 years of age or older with reversible obstructive airways disease, including patients with symptoms of nocturnal asthma, who require regular treatment with inhaled, short-acting, beta2 agonists. It is not indicated for patients whose asthma can be managed by occasional use of inhaled, short-acting, beta2agonists. For the acute prevention of exercise-induced bronchospasm (EIB) in adults and children 12 years of age or older, when administered on an occasional, as needed basis. For the long-term, twice-daily (morning and evening) administration in the maintenance treatment of bronchoconstriction in patients with COPD, including chronic bronchitis and emphysema |
| HALOTHANE | For the induction and maintenance of general anesthesia. |
| ILOPROST | For the treatment of pulmonary arterial hypertension (World Health Organization[WHO] group I) in patients with New York Heart Association (NYHA) class III or IV symptoms. |
| INSULIN RECOMBINANT HUMAN | For the treatment of adult patients with diabetes mellitus for the control of hyperglycemia. |
| ISOETHARINE HYDROCHLORIDE | For bronchial asthma and reversible bronchospasm that occurs with bronchitis and emphysema. |
| | |
| ISOFLURANE | For induction and maintenance of general anesthesia. Adequate data have not been developed to establish its application in obstetrical anesthesia. |
| ISOPROTERENOL HYDROCHLORIDE | For mild or transient episodes of heart block that do not require electric shock or pacemaker therapy. For serious episodes of heart block and Adams-Stokes attacks (except when caused by ventricular tachycardia or fibrillation). For use in cardiac arrest until electric shock or pacemaker therapy, the treatments of choice, is available. For bronchospasm occurring during anesthesia. As an adjunct to fluid and electrolyte replacement therapy and the use of other drugs and procedures in the treatment of hypovolemic and septic shock, low cardiac output (hypoperfusion) states, congestive heart failure, and cardiogenic shock. |
| LEVALBUTEROL HYDROCHLORIDE | For the treatment or prevention of bronchospasm in adults, adolescents, and children 6 years of age and older with reversible obstructive airway disease. |
| METAPROTERENOL SULFATE | In the treatment of asthma and bronchitis or emphysema when a reversible component is present in adults and for the treatment of acute asthmatic attacks in children 6 years of age or older. |
| METHACHOLINE CHLORIDE | For the diagnosis of bronchial airway hyperreactivity in subjects who do not have clinically apparent asthma. |
| | |
| MOMETASONE FUROATE | For the maintenance treatment of asthma as prophylactic therapy in patients 12 years of age and older. Mometasone also is indicated for asthma patients who require oral corticosteroid therapy, where adding mometasone therapy may reduce or eliminate the need for oral corticosteroids. |
| NEDOCROMIL SODIUM | For maintenance therapy in the management of adult and pediatric patients 6 years and older with mild to moderate asthma. |
| NITRIC OXIDE | Nitric oxide, in conjunction with ventilatory support and other appropriate agents, is indicated for the treatment of term and near-term (greater than 34 weeks) neonates with hypoxic respiratory failure associated with clinical or echocardiographic (ECG) evidence of pulmonary hypertension, where it improves oxygenation and reduces the need for extracorporeal membrane oxygenation. |
| PENTAMIDINE ISETHIONATE | For the prevention of Pneumocystis carinii pneumonia(PCP) in high-risk, HIV-infected patients defined by 1 or both of the following criteria: A history of 1 or more episodes of PCP. A peripheral CD4+ (T4 helper/inducer) lymphocyte count less than or equal to 200/mm3. |
| PENTETATE CALCIUM TRISODIUM | Pentetate calcium trisodium is indicated for treatment of individuals with known or suspected internal contamination with plutonium, americium, or curium to increase the rates of elimination. |
| | |
| PENTETATE ZINC TRISODIUM | For treatment of individuals with known or suspected internal contamination with plutonium, americium, or curium to increase the rates of elimination. |
| PIRBUTEROL ACETATE | For the prevention and reversal of bronchospasm in patients 12 years of age and older with reversible bronchospasm including asthma. It may be used with or without concurrent theophylline and/or corticosteroid therapy. |
| RIBAVIRIN | For the treatment of hospitalized infants and young children with severe lower respiratory tract infections due to respiratory syncytial virus (RSV). |
| SALMETEROL XINAFOATE | For long-term, twice daily (morning and evening) administration in the maintenance treatment of asthma and in the prevention of bronchospasm in patients 4 years of age and older with reversible obstructive airway disease, including patients with symptoms of nocturnal asthma. |
| SEVOFLURANE | Induction and maintenance of general anesthesia in adults and children for inpatient and outpatient surgery |
| TETRAHYDROCANNABINOL | For the treatment of anorexia associated with weight loss in patients with acquired immune deficiency syndrome (AIDS); and nausea and vomiting associated with cancer chemotherapy in patients who have failed to respond adequately to conventional antiemetic treatments. |
| | |
| TIOTROPIUM BROMIDE MONOHYDRATE | Alone or with other bronchodilators, especially beta adrenergics, as a bronchodilator for maintenance treatment of bronchospasm associated with COPD, including chronic bronchitis and emphysema. |
| TOBRAMYCIN | For the management of cystic fibrosis patients with P. aeruginosa. |
| TRIAMCINOLONE ACETONIDE | In the maintenance treatment of asthma as prophylactic therapy; for asthma patients who require systemic corticosteroids, where adding an inhaled corticosteroid may reduce or eliminate the need for the systemic corticosteroids. |
| ZANAMIVIR | For treatment of uncomplicated acute illness caused by influenza A and B virus in adults and children at least 7 years of age who have been symptomatic for no more than 2 days. |

In addition to the above-listed drugs already FDA approved for pulmonary delivery, other drugs referenced for possible pulmonary delivery by the disclosed methods include, but are not limited to, those provided in Table 2 below.

**Table 2:**

| | | |
|---|---|---|
| 2-pentenylpenicillin | a/t/s, erythromycin | abarelix |
| abbokinase | abelcet | abilify |
| abraxane | abreva | accolate |
| accuneb | accupril | accuretic |
| accutane | acebutolol | acebutolol hydrochloride |
| aceclofenac | aceon | acephen |
| acetadote | acetaminophen | acetaphenazine |
| acetasol | acetazolamide | acetazolamide sodium |
| acetic acid | acetohexamide | acetophenazine |
| acetylcysteine | acilac | aciphex |
| aclasta | aclovate | acomplia |
| acthrel | actigall | actimmune [interferon gamma-1b] |
| actiq | activase [alteplase] | activella |
| actonel | actoplus met | actos |
| actraphane | actrapid | actron |
| acular | acutect | acyclovir |
| acyclovir sodium | aczone | adagen |
| adalat cc | adderall | ademetionine |
| adenocard | adenoscan | adenosine |
| adinazolam | adipex-p | adrafinil |
| adriamycin pfs | adrucil | advair |
| advair diskus | advate | advicor |
| advil | aerius | aerobid |
| afrinol | agenerase | aggrastat |
| aggrenox | Agrylin | Ahnotriptan |
| a-hydrocort | akbeta | Akineton |
| akne-mycin | akpentolate | Akpro |
| Aktob | ala-cort | Alamast |
| ala-scalp | alavert | albalon |
| albenza | albumin iodinated i-125 serum | albumin iodinated i-131 serum |
| albumin, human | albuterol | albuterol sulfate |
| alcaine | alclometasone dipropionate | alcohol |
| aldactazide | aldactone | aldara |
| aldesleukin (proleukin) | aldoril | aldurazyme |
| alendronate | alertec | alesse |
| aleve | alfentae | alfentanil |
| alfentanil hcl | alfentanil hydrochloride | alglucerase |
| alimta | alinia | alizapride |
| alkeran | allay | allegrae |
| allex | Allopurinol | allopurinol sodium |
| Allylprodine | Alminoprofen | almotriptan |
| alocril | alomide | aloprimm |
| alora | aloxi | alperopride |
| alphagan p | alphaprodine | alphatrex |
| alpidem | alprazolam | alprostadil |
| alrex | alseroxlon | altace |
| altoprev | alupent | alvesco |
| amantadine | amantadine hydrochloride | amaryl |
| ambenyl | ambien | ambirix |
| ambisome | ambrisentan | amcinonide |
| amerge | amesergide | a-methapred |
| amfenac | amicar | amidate |
| amifostine | amikacin | amikacin sulfate |
| amikin | amiloride hydrochloride | amino acids |
| aminoacetic acid | aminocaproic acid | aminohippurate sodium |
| aminophylline | aminopropylon | aminosyn |
| amiodarone | amiodarone hcl | amiodarone hydrochloride |
| amisulpride | amitriptyline | amitriptyline hydrochloride |
| amixetrine | amlexanox | amlodipine |
| amlodipine besylate | ammonaps | ammonium chloride |
| ammonium lactate | Ammonul | amnesteem |
| Amoxapine | Amoxicillin | Amoxil |
| Amperozide | Amphadase | Amphenidone |
| Amphetamine | ampho b | ampho b |
| amphotec | amphotericin b | ampicillin |
| ampicillin and sulbactam | ampicillin sodium | ampicillin sodium/sulbactam sodium |
| ampicillin trihydrate | ampicillin/sulbactam | amrinone lactate |
| amylpenicillin | anadrol-50 | anafranil |
| anagrelide hydrochloride | anagryd | anandron |
| anaprox | ancef | ancobon |
| androderm | androgel | android |
| andropinirole | an-dtpa | anectine |
| anestacon | anexsia | angeliq |
| angiomax | angiox inn | anidulafungin |
| anileridine | anileridine | ansaid |
| anspor | an-sulfur colloid | antabuse |
| antara (micronized) | antivert | antizol |
| anturane | anusol hc | anzemet |
| anzemete | apazone | aphthasol |
| apidra | apo-cilazapril/hctz | apo-digoxin |
| apo-etidronate | apo-feno-super | apo-flecainide |
| apokyn | apo-levetiracetam | apo-medroxy |
| apo-meloxicam | apo-methotrexate | apo-metoprolol sr |
| apo-midodrine | apo-mirtazapine | apomorphine |
| apomorphine hydrochloride | apomorphinediacetate | apo-omeprazole |
| apo-ondansetron | apo-oxcarbazepine | apo-ramipril |
| apo-ranitidine | apo-risperidone | apo-sumatriptan |
| apo-topiramate | apresazide | aprotinin bovine |
| aprovel | aptivus | aquamephyton |
| aquasol a | aralen | aramine |
| aranelle | aranesp | arava |
| aredia | arestin | arestin microspheres |
| argatroban | argatroban | arginine hydrochloride |
| aricept | ariclaim | arimidex |
| aristocort a | aristospan | arixtra |
| aromasin | arranon | arsenic trioxide |
| arthrotec | articaine hydrochloride/epinephrine | asacol |
| asmanex twisthaler | aspirin | astelin |
| astramorph pf | atacand | atarax |
| atenolol | ativan | atracurium besylate |
| atridox | atropen | atropine sulfate |
| atropine sulfate/edrophonium chloride | atrovent | atrovent |
| atryn | attenade | augmentin |
| avagard | avage | avaglim |
| avalide | avandamet | avandaryl |
| avandia | avapro | avastin |
| avelox | aventyl hydrochloride | aviane-28 |
| avinza | avita | avodart |
| avonex | axert | axid |
| axura | aygestin | azacitidine |
| azactam | azacyclonol | azasan |
| azasetron | azatadine | azathioprine |
| azathioprine sodium | azdone | azelex |
| azidocillin | azilect | azithromycin |
| azmacort | azomyr | azopt |
| aztreonam | azulfidine | baciim |
| bacille calmette-guérin | baci-rx | bacitracin |
| bacitracin zinc | baclofen | bacteriostatic sodium chloride |
| bacteriostatic water | bactocill | bactrim |
| bactroban | bal | balziva |
| baraclude | baros | bayer |
| beclomethasone dipropionate | beconase aq | benactyzine |
| benadryl | benazepril hydrochloride | benefix |
| benicar | benmoxine | benoquin |
| benoxaprofen | benperidol | benserazide |
| bentyl | benzaclin | benzamycin |
| benzonatate | benzoyl peroxide | benzpiperylon |
| benzquinamide | benzquinamide hydrochloride | benztropine |
| benztropine mesylate | benzydramine | benzylmorphine |
| benzylpenicillin | beractant | beromun |
| bertezomib | beta-2 | betadine |
| betaferon | betagan | betamethasone acetate |
| betamethasone dipropionate | betamethasone sodium phosphate | betamethasone valerate |
| betapace | betaseron [interferon beta-1 b] | beta-val |
| betaxolol | betaxolol hydrochloride | bethanechol chloride |
| betimol | betoptic | bextra |
| bexxar [tositumomab] | bezitramide | biaxin |
| bicillin | bicnu | bidil |
| biltricide | binedaline | bioscrub |
| biperiden | biphentin | bisoprolol fumarate |
| bitolterol | bitolterol mesylate | bivalirudin |
| blenoxane | bleomycin | bleomycin sulfate |
| bleph | blephamide | blocadren |
| bolusacplus | bondenza | bondronat |
| boniva | bontril | bonviva |
| botox [botulinum toxin type a] | branchamin | bravelle |
| breathtek ubt | brethine | bretylium tosylate |
| brevibloc | brevicon | brevital sodium |
| brian care | brimonidine tartrate | bristacycline |
| brofaromine | bromfed-dm | bromfenac |
| bromisovalum | bromocriptine | bromocriptine mesylate |
| bromopride | bromperidol | brompheniramine |
| brompheniramine maleate | broncho saline | bronitin mist |
| brucine | bss | bucet |
| buclizine | budesonide | budesonide; formoterol fumarate |
| budipine | bufexamac | bumetanide |
| bumex | buphenyl | bupivacaine hydrochloride |
| bupivacaine hydrochloride/epinephrine | bupivacaine hydrochloride/epinephrine bitartrate | buprenex |
| buprenorphine | buprenorphine hydrochloride | buprenorphine hydrochloride/naloxone hydrochloride |
| bupropion | bupropion hydrochloride | buramate |
| busilvex | buspar | buspirone |
| buspirone hydrochloride | busulfan | busulfex |
| butabarbital | butaclamol | butal compound |
| butalbital | butapap | butaperazine |
| butisol sodium | butorphanol | butorphanol tartrate |
| butriptyline | byetta | cabergoline |
| caduet | caelyx | cafcit |
| cafergot | caffeine | caffeine citrate |
| calan | calcijex | calcimar |
| calcitonin, salmon | calcitriol | calcium chloride |
| calcium disodium versenate | calcium gluconate | calcium-n-carboamoylaspartate |
| calfactant | camila | campath [alemtuzumab] |
| campral | camptosar | canasa |
| cancidas | cannobinoids | cantil |
| capastat sulfate | capital and codeine | capitrol |
| capoten | capozide | cap-profen |
| capreomycin sulfate | captodiamine | captopril |
| capuride | carac | carafate |
| carbaglu | carbamazepine | carbastat |
| carbatrol | carbcloral | carbenicillin |
| carbidopa | carbilev | carbinoxamine maleate |
| carbiphene | carbocaine | carboplatin |
| carboprost tromethamine | carbromal | cardene |
| cardene sr | cardiogen-82 | cardiolite |
| cardioplegic | cardizem | cardura |
| carfecillin | carindacillin | carisoprodol |
| carmol hc | carmustine | carnitor |
| caroxazone | carphenazine | carpipramine |
| carprofen | carteolol hydrochloride | cartia xt |
| casodex | caspofungin | caspofungin acetate |
| caspofungin msd | cataflam | catapres |
| cataprese | cathflo activase [alteplase] | caverject |
| cea-scan | cedax | ceenu |
| cefaclor | cefadroxil | cefazolin |
| cefazolin sodium | cefepime hydrochloride | cefinetazole |
| cefizox | cefmetazole | cefobid |
| cefotan | cefotaxime | cefotaxime sodium |
| cefoxitin | cefoxitin sodium | cefpodoxime proxetil |
| cefprozil | ceftazidime | ceftazidime sodium |
| ceftin | ceftizoxime sodium | ceftriaxone |
| ceftriaxone sodium | cefuroxime | cefuroxime axetil |
| cefuroxime sodium | cefzil | celebrex |
| celestone | celestone soluspan | celexa |
| cellcept | celontin | cenestin |
| centany | cephacetrile | cephalexin |
| cephaloglycin | cephaloridine | cephalosporin c |
| cephalosporins | cephalotin | cephamycin a |
| cephamycin b | cephamycin c | cephamycins |
| cepharin | cephradine | ceprotin |
| ceptaz | cerebyx | ceredase |
| ceretec | cerezyme | cericlamine |
| cerubidine | cervidil | cetacort |
| cetamide | cetrizine | cetrorelix |
| cetrotide | champix | chemet |
| chg scrub) | children's advil | children's elixsure |
| children's ibuprofen | children's motrin | children's motrin |
| chirhostim | chloralbetaine | chloramphenicol |
| chloramphenicol sodium succinate | chloraprep | chlorascrub |
| chlordiazepoxide | chlorhexidine gluconate | chlorobutinpenicillin |
| chloromycetin | chloroprocaine hydrochloride | chloroquine phosphate |
| chlorothiazide | chlorothiazide sodium | chlorpheniramine |
| chlorpromazine | chlorpromazine hydrochloride | chlorpromazine hydrochloride intensol |
| chlorpropamide | chlorprothixene | chlorthalidone |
| chlor-trimeton | chlorzoxazone | cholac |
| choledyl sa | cholestagel | cholestyramine |
| choletec | choline | cholografin meglumine |
| choriogonadotropin alfa | chorionic gonadotropin | chromic chloride |
| chromic phosphate, p-32 | chromitope sodium | cialis |
| ciclopirox | cida-stat | cidofovir |
| cilazaprol | cilostazol | ciloxan |
| cimetidine | cimetidine hydrochloride | cinchophen |
| cinmetacin | cinnarizine | cipralex |
| cipramadol | cipro | ciprodex |
| ciprofloxacin | ciprofloxacin hydrochloride | ciprofloxacin, levofloxacin |
| cisatracurium besylate | cis-mdp | cisplatin |
| cis-pyro | citalopram | citalopram hydrobromide |
| citanest | cladribine | claforan |
| claravis | clarinex | clarithromycin |
| claritin | clebopride | clemastine |
| clemastine fumarate | cleocin | cleocin hydrochloride |
| cleocin phosphate | cleocin t | climara |
| clinda-derm | clindagel | clindamycin |
| clindamycin hydrochloride | clindamycin phosphate | clinda-t |
| clindesse | clindets | clinimix |
| clinisol | clinoril | clobenzepam |
| clobetasol propionate | clobex | clocapramine |
| cloderm | clofarabine | clofibrate |
| clolar | clomacran | clometacin |
| clometocillin | clomid | clomiphene citrate |
| clomipramine | clomipramine hydrochloride | clonazepam |
| clonidine | clonidine hydrochloride | clonitazene |
| clonixin | clopenthixol | clopriac |
| clorazepate dipotassium | clorpres | clospirazine |
| clothiapine | clotrimazole | clovoxamine |
| cloxacillin | cloxacillin sodium | cloxapen |
| clozapine | clozaril | co bicalutamide |
| co cilazapril | co fluconazole | co fosinopril |
| co ipra-sal | co risperidone | co salbut-iprat inhalation solution |
| co topiramate | coaprovel | codeine |
| codeprex | codrix | cogentin |
| co-gesic | cognex | co-lav |
| colazal | colestid | colgate |
| colistimethate | colistimethate sodium | colocort |
| col-probenecid | coly-mycin m | coly-mycin s |
| colyte | combipatch | combivent |
| combivir | combunox | commit |
| compazine | competact | compro |
| comtan | comtess | concerta |
| concertae | condylox | conivaptan hydrochloride |
| conray | constilac | constulose |
| copasys copegus [peginterferon alfa-2a] | copaxone | copegus |
| copper | cordarone | cordran |
| coreg | corgard | corlentor |
| corlopam | cormax | corphed |
| cortef | cortenema | corticorelin ovine triflutate |
| corticotropin | cortifoam | cortifoam, hydrocortisone acetate |
| cortisone acetate | cortisporin | cortrosyn |
| corvert | corzide | co-sertraline |
| cosmegen | cosopt | cosyntropin |
| cotinine | cotrim | cotronak |
| coumadin | covera-hs | coversyl |
| cozaar | crestor | crinone |
| crixivan | crolom | cromolyn sodium |
| crotan | cryselle | c-solve-2 |
| cubicin | cupric chloride | cuprimine |
| curosurf | cutivate | cyamemazine |
| cyanocobalamin | cyclacillin | cyclessa |
| cyclizine | cyclobenzaprine | cyclobenzaprine hydrochloride |
| cyclocort | cyclogyl | cyclomen |
| cyclomydril | cyclopentolate hydrochloride | cyclophosphamide |
| cyclosporin a | cyclosporine | cyklokapron |
| cymbalta | cyproheptadine | cyproheptadine hydrochloride |
| cystadane | cystagon | cysto-conray ii |
| cystografin | cystografin dilute | cytadren |
| cytarabine | cytomegalovirus immune globulin (cmv-igiv) | cytomel |
| cytosar-u | cytotec | cytovene iv |
| cytoxan | d.h.e. 45 | dacarbazine |
| dactinomycin | dalmane | dalteparin sodium |
| danazol | dantrium | dantrolene sodium |
| dapsone | daptomycin | daquiran |
| daraprim | darbepoetin alfa (aranesp) | darvocet |
| darvon | datscan | daunorubicin citrate |
| daunorubicin hydrochloride | daunoxome | daypro |
| ddavp | ddavp melt | decadron |
| declomycin | deferoxamine mesylate | definity |
| delatestryl | delestrogen | delflex |
| delsym | demadex | demeclocycline hydrochloride |
| demerol | demser | demulen |
| denavir | dendrid | depacon |
| depakene | depakote | depen |
| depocyt | depocyte | depodur |
| depo-estradiol | depo-medrol | depo-provera |
| depo-subq provera 104 | depo-testosterone | deprenyl |
| dermabet | derma-smoothe/fs | dermatop |
| desferal | desflurane | desipramine |
| desipramine hydrochloride | desirudin recombinant | desmopressin acetate |
| desogen | desogestrel | desonide |
| desowen | desoximetasone | desoxyn |
| desyrel | detrol | dexamethasone |
| dexamethasone | dexamethasone intensol | dexamethasone sodium phosphate |
| dexasporin | dexchlorpheniramine maleate | dexedrine |
| dexfenfluramine | dexferrum | dexmedetomidine |
| dexrazoxane | dexrazoxane hydrochloride | dextroamp saccharate |
| dextroamphetamine | dextroamphetamine sulfate | dextromoramide |
| dextropropoxyphene | dextrose | dextrostat |
| diabeta | diabinese | dialyte |
| dialyte concentrate | diamorphine | diamox |
| dianeal | diastat | diastat acudial |
| diatrizoate meglumine | diatrizoate sodium | diazepam |
| diazepam intensol | diazoxide | dibenzyline |
| diclofenac | dicloxacillin | dicloxacillin sodium |
| dicyclomine hydrochloride | didanosine | didrex |
| didronel | differin | diflorasone diacetate |
| diflucan | diflunisal | digoxin |
| dihydrocodeine | dihydroergokryptine | dihydroergotamine |
| dihydroergotamine mesylate | dilacor | dilantin |
| dilatrate-sr | dilaudid | dilor |
| dilt-cd, | diltiazem | diltiazem hydrochloride |
| dimenhydrinate | dimercaprol | dimethyl sulfoxide |
| diovan | dipentum | diphenhydramine |
| diphenhydramine hydrochloride | diphenicillin | diphenidol |
| diphenoxylate | dipipanone | dipivefrin hydrochloride |
| diprivan | diprolene | dipyridamole |
| disophrol | disopyramide phosphate | dispermox |
| disulfiram | ditropan | diupres- |
| diuril | dobutamine hydrochloride | dobutrex |
| docetaxel | dolasetron mesylate monohydrate | dolasetronmethanesulfonate |
| dolobid | dolophine hydrochloride | dom-alendronate |
| dom-anagrelide | dom-bicalutamide | dom-citalopram |
| dom-doxycycline | domeridone | dom-hydrochlorothiazide |
| dom-mirtazapine | dom-ondanssetron | dom-risperidone |
| dom-simvastatin | dom-ursodiol c | dopamine hydrochloride |
| dopram | doral | dornase alfa |
| doryx | dostinex | dosulepin |
| dovonex | doxacalciferol | doxapram hydrochloride |
| doxazosin mesylate | doxepin | doxepin hydrochloride |
| doxil | doxorubicin | doxorubicin hydrochloride |
| doxy | doxycycline | doxycycline hyclate |
| doxylamine | doxylamine succinate | draximage mdp |
| dricort | drisdol | drixoral |
| dronabinol | droperidol | droprenilamin hcl |
| droxia | dtic-dome | dtpa |
| duac | dukoral | duloxetine |
| duo trav | duocaine | duoneb |
| duotrav | duraclon | duragesic |
| duragesic mat | duramorph | duricef |
| duvoid | dyazide, hydrochlorothiazide | dynacin |
| dynacirc | dyna-hex | dynastat |
| dynepo | dyrenium | e.e.s. erythromycin ethylsuccinate |
| e-base | ebixa | ec-naprosyn |
| econazole nitrate | econopred | edecrin |
| edetate calcium disodium | edetate disodium | edex |
| edrophonium chloride | effexor | efidac |
| efudex | e-glades | eldepryl |
| elestat | eletriptan | elidel |
| eligard | elimite | eliprodil |
| elitek [rasburicase] | elixophyllin | ellence |
| elliotts b solution | elmiron | elocon |
| eloxatin | elspar [asparaginase] | emadine |
| embeline | emcyt | emend |
| emete-con | emla | emselex |
| emtriva | e-mycin | enablex |
| enalapril | enalapril maleate | enalaprilat |
| enbrel | enciprazine | endosol |
| endrate | enduron | enflurane |
| enfuvirtide | enlon | enoxaparin sodium |
| enpresse-28 | entacapone | entocort |
| entonox | enulose | ephedrine |
| epifoam | epinephrine | epipen |
| epirubicin hydrochloride | epitol | epivir |
| epoetin alfa (procrit) | epogen [epoetin alfa] | epoprostenol sodium |
| eptastigmine | eptifibatide | epzicom |
| equagesic | equetro | erbitux |
| ergolinepramipexole | ergoloid mesylates | ergomar |
| ergotamine | ergotamine tartrate | eribitux [cetuximab] |
| errin | ertaczo | ertapenem sodium |
| eryc | erycette | eryderm |
| erygel | eryped | ery-tab |
| erythra-derm | erythrocin | erythrocin stearate |
| erythromycin | erythromycin estolate | erythromycin ethylsuccinate |
| erythromycin lactobionate | erythromycin stearate | eryzole |
| esgic-plus | esidrix | eskalith |
| esmolol hydrochloride | e-solve 2 | esomeprazole sodium |
| estazolam | estrace | estraderm |
| estradiol | estradiol cypionate | estradiol valerate |
| estrasorb | estring | estrogel |
| estrogens, conjugated | estrone | estropipate |
| estrostep fe | etamiphyllin | etaqualone |
| ethacrynate sodium | ethambutol | ethambutol hydrochloride |
| ethamolin | ethanolamine oleate | ethiodol |
| ethmozine | ethoheptazine | ethosuximide |
| ethrane | ethyol | etidronate disodium |
| etodolac | etomidate | etopophos |
| etoposide | etoposide phosphate | eulexin |
| eurax | evalose | evista |
| evocline | evoltra | evoxac |
| evra | excedrin | exelderm |
| exelon | exenatide synthetic | exidine |
| exjade | exjade | exosurf neonatal |
| extended phenytoin sodium | extraneal | extra-strength aim |
| exubera | e-z prep | ezetrol |
| fabrazyme | factive | factor ix complex (konyne 80, profilnine heat-treated, proplex sx-t, proplex-t) |
| factor vii (novoseven, niastase) | factor viii (alphanate, hemofil m, humate-p, koate-hp, koate-hs, monoclate-p) | factor xi (hemoleven, factor xi concentrate [bpl]) |
| famotidine | famvir | fansidar |
| fareston | faslodex | fasturtec |
| fazaclo odt | felbatol | feldene |
| felodipine | femara | femhrt |
| femring | femstat 3 | femtrace |
| fendrix | fenfluramine | fenofibrate |
| fenoldopam mesylate | fenomax | fenoprofen calcium |
| fentanyl | fentanyl citrate | feridex i.v. |
| ferriprox | ferrlecit | ferumoxides |
| fexofenadine | fexofenadine hydrochloride | fientanyl |
| filgrastim (neupogen) | finacea | fioricet |
| fiorinal | flagyl | flagystatin |
| flarex | flavoxate hydrochloride | flecainide acetate |
| flesinoxan | flexeril | flolan |
| flomax | flonase | florinef |
| florone | flovent | flovent hfa |
| floxin | fluconazole | fluconazole |
| fludara | fludarabine phosphate | fludeoxyglucose |
| fludeoxyglucose f-18 | fludrocortisone acetate | flumadine |
| flumazenil | flunisolide | fluocinolone acetonide |
| fluocinonide | fluor-op | fluoroplex |
| fluorouracil | fluotic | fluoxetine |
| fluoxetine hydrochloride | fluoxymesterone | flupenthixol |
| fluphenazine | fluphenazine decanoate | fluphenazine hydrochloride |
| flupirtine | flurazepam | flurazepam hydrochloride |
| flurbiprofen | flurbiprofen sodium | fluspirilene |
| flutamide | fluticasone propionate | fluvoxamine |
| fluvoxamine maleate | fluxid | fml |
| foamcoat | focalin | folic acid |
| folicet | follistim aq | follitropin alfa/beta |
| fomepizole | fondaparinux sodium | foradil |
| forane | forcaltonin | formoterol fumarate |
| forsteo | fortamete | fortaz |
| forteo | fortical | fortovase |
| fosamax | fosavance | foscan |
| foscarnet sodium | foscavir | fosinopril |
| fosinopril sodium | fosphenytoin sodium | fosrenol |
| fragmin | fraxiparine and fraxiparine forte | freamine |
| frova | frovatriptan | fs shampoo |
| fudr | fulvestrant | fulvicin-u/f |
| fungizone | furadantin | furosemide |
| fuzeon | gabapentin | gabitril |
| gadobenate dimeglumine | gadodiamide | gadopentetate dimeglumine |
| gadoteridol | gadoversetamide | galanthamine |
| gallium citrate ga 67 | gallium nitrate | galzin |
| ganciclovir | ganciclovir sodium | ganfort |
| ganirelix acetate | ganite | gantrisin pediatric |
| gardasil | gastrocrom | gastrografin |
| gastromark | gaviscon | gd-amlodipine |
| gd-atorvastatin | gd-azithromycin | gd-fluconazole |
| gd-gabapentin | gd-gemfibrozil | gd-sertraline |
| gemcitabine hydrochloride | gemfibrozil | gemtuzumab ozofamicin |
| gemzar | gen-alendronate | gen-azithromycin |
| gencept | gen-cilazapril | gen-domperidone |
| generlac | gen-glimepiride | gengraf |
| gen-meloxicam | genoptic | genotropin |
| gen-pravastatin | gen-risperidone | gentacidin |
| gentak | gentamicin | gentamicin sulfate |
| gen-warfarin | gen-xene | geocillin |
| geodon | gepirone | geref |
| gerimal | ghrelin | gleevec |
| gliadel | gliclazide | glimepiride |
| glipizide | glivec | glofil-125 |
| glucagen | glucagon | glucobay |
| glucophage | glucotrol | glucovance |
| glumetza | glustin | glutathione |
| glyburide | glycine | glycolax |
| glycopyrrolate | glynase | glyset |
| gmd - azithromycin | gmd-sertraline | go-evac |
| golytely | gonadotropic, chorionic | gonal-f |
| granisetron | granisetron hydrochloride | grifulvin v |
| griseofulvin | gris-peg | growth hormone |
| guanabenz acetate | guanfacine hydrochloride | guanidine hydrochloride |
| gynazole-1 | gyne-lotrimin | gynix |
| gynodiol | h.p. acthar gel | habitrol |
| halcion | haldol | halflytely |
| halobetasol propionate | halog | haloperidol |
| haloperidol decanoate | haloperidol lactate | haloperidole |
| halotestin | halothane | hbvaxpro |
| hectorol | helicobacter test infai | helidac |
| heliox | helixate nexgen | hemabate |
| hepacare | heparin lock flush | heparin sodium |
| hepatamine | hepatasol | hepatitis b immune globulin (bayhep b, hepagam b) |
| hepatolite | hepflush-10 | hep-lock |
| hepsera | heptalac | heptylpenicillin |
| herceptin | herplex | hetacillin |
| hexabrix | hexadrol | hexalen |
| hexavac | hibiclens | hibistat |
| hi-cor | hiprex | hivid |
| hms | homatroprine methylbromide | humalog |
| humaspect | humatin | humatrope |
| humira | humulin | hycamtin |
| hycodan | hydase | hydergine |
| hydralazine hydrochloride | hydra-zide | hydrea |
| hydrocet | hydrochlorothiazide | hydrocodone bitartrate |
| hydrocortisone | hydrocortisone sodium succinate | hydroflumethiazide |
| hydrogenated ergot alkaloids | hydromorphone | hydromorphone hydrochloride |
| hydro-ride | hydroxocobalamin | hydroxychloroquine sulfate |
| hydroxyurea | hydroxyzine | hydroxyzine hydrochloride |
| hydroxyzine pamoate | hylenex recombinant | hyoscine |
| hypaque | hypaque oral powder | hyperstat |
| hy-phen | hytone | hytrin |
| hyzaar | ibandronate | ibandronic acid roche |
| ibu | ibuprofen | ibuprofen lysine (neoprofen) |
| ibuprohm | ibutilide fumarate | ic-green |
| idamycin pfs | idarubicin hydrochloride | idazoxan |
| ifex/mesnex kit | ifosfamide | iletin ii |
| iloprost | imdur | imiglucerase |
| imipenem/cilastatin, meropenem, ertapenem | imipramine | imipramine hydrochloride |
| imitrex | immune globulin (baygam, vivaglobin) | imodium |
| imovane | imuran | inamrinone lactate |
| inapsine | increlex | indapamide |
| inderal la | inderide | indiclor |
| indium | indium in 111 chloride | indium in 111 oxyquinoline |
| indium in 111 pentetate disodium | indium in 111 pentetreotide | indocin |
| indocyanine green | indo-lemmon | indomethacin |
| indomethacin sodium | indomethegan | indoprofen |
| inductos | infanrix hepb | infanrix hexa |
| infanrix penta | infants' feverall | infasurf |
| infed | infergen | inflamase |
| infumorph | infuvite | innofem |
| innohep | innopran xl | inomax |
| inpersol-Ic/Im | inspra | insulatard |
| insulin | insulin (recombinant human) | insulin aspart recombinant |
| insulin detemir recombinant | insulin glargine recombinant | insulin lispro protamine recombinant |
| insulin purified pork | insulin recombinant human | insuman |
| intal | integrilin | interferon alfa-2b (intron, pegintron, pegasys) |
| intralipid | intrinsa | introna |
| invagesic | invanz | inversine |
| invirase | iobenguane sulfate i 131 | iodipamide meglumine |
| iodixanol | iodotope | iohexol |
| ionamin | ionosol | ionsys |
| iopamidol | iopidine | iopromide |
| iosat | iothalamate meglumine | iothalamate sodium |
| iothalamate sodium i-125 | ioversol | ioxaglate meglumine |
| ioxaglate sodium | ioxilan | iplex |
| ipratropium bromide | iprivask | iproniazid |
| ipsapiraone | iquix | iressa |
| irinotecan hydrochloride | iron dextran | iron sucrose |
| iscover | ismo | isocaine hydrochloride |
| isocarboxazid | isoetharine hydrochloride | isoflurane |
| isolyte | isometheptene | isoniazid |
| isoproterenol | isoproterenol bitartrate | isoproterenol hydrochloride |
| isoptin | isopto cetamide | isordil |
| isosorbide dinitrate | isosorbide mononitrate | isosulfan blue |
| isotonic gentamicin sulfate | isovue | isradipine |
| istalol | isuprel | itraconazole |
| ivadal | ivy block | ixense |
| jantoven | jeanatope | joi-risperidone |
| junel | k+10 | k+8 |
| kadian | kaletra | kanamycin |
| kanamycin sulfate | kantrex | kaon cl-10 |
| kariva | karvea | karvezide |
| kayexalate | k-dur | keflex |
| kefurox | kefzol | kelnor |
| kemadrin | kemstro | kenacort |
| kenalog | kentera | kepivance |
| keppra | kerlone | ketalar |
| ketamine | ketamine hydrochloride | ketek |
| ketoprofen | ketoprofen | ketorolac |
| ketorolac tromethamine | ketotifen | ketozole |
| kineret | kinevac | kinzalkomb |
| kinzalmono | kionex | kiovig |
| kitanserin | kivexa | klaron |
| klonopin | klor-con | klotrix |
| kogenate bayer | k-tab | kudeq |
| kytril | labetalol hydrochloride | lac-hydrin |
| lacrisert | lactated ringer's | lactulose |
| I-alphaacetylmethadol | lamictal | lamisil |
| laniazid | lanorinal | lanoxicaps |
| lanoxin | lansoprazole | lantus |
| largactil | lariam | larotid |
| laryng-o-jet kit | lasix | laxilose |
| lazabemide | leflunomide | lente iletin ii (pork) |
| lepirudin recombinant | leptin | lescol |
| lesopitron | lessina-28 | leucovorin calcium |
| leukeran | leukine [sargramostim] | leukoscan |
| leuprolide acetate | leustatin | levalbuterol hydrochloride |
| levaquin | levatol | levemir |
| levitra | levlite | levobunolol hydrochloride |
| levocarnitine | levodopa | levo-dromoran |
| levofloxacin | levolet | levonorgestrel |
| levophed | levora | levorphanol |
| levorphanol tartrate | levo-t | levothroid |
| levothyroxine sodium | levoxyl | levulan |
| levviax | lexapro | lexiva |
| lexxel | librium | lidex |
| lidocaine | lidocaine hydrochloride | lidocaine viscous |
| lidocaton | lidoderm | lidopen |
| lidosite topical system kit | lignospan | limbitrol |
| lincocin | lincomycin hydrochloride | lindane |
| linezolid | lioresal | liothyronine sodium |
| lipitor | liposyn | lisinopril |
| lisinopril | lisuride | litak |
| lithium carbonate | lithium citrate | lithobid |
| lithostat | livensa | livostin |
| lo/ovral-28 | locacorten vioform | locholest |
| locoid | Iodine | lodosyn |
| loestrin | lofentanil | lofepramine |
| lomotil | lomustine | loniten |
| lonox | loperamide hydrochloride | lopid |
| loprazolam | lopressor | loprox |
| lopurin | lorabid | loratidine |
| lorazepam | lorazepam intensol, lorazepam | lorcet-hd |
| lorelco | lorexan | lorezepam |
| lortab | lotemax | lotensin |
| lotrel | lotrimin | lotrisone |
| lotronex | lovastatin | lovenox |
| low-ogestrel | loxapine | loxapine succinate |
| loxitane | lozol | Ita ii kit |
| lufyllin | lumigan | luminity |
| lunesta | lupron | lutropin alfa |
| luveris | luxiq | lymphazurin |
| lyophilized cytoxan | lyrica | lysodren |
| m.t.e.-4/m.t.e -6 | m.v.i. i. | mabcampath |
| mabthera | macrobid | macrodantin |
| macrotec | macugen | magnesium sulfate |
| magnevist | malarone | manganese chloride |
| mannitol | maprotiline hydrochloride | maprotoline |
| marcaine | marcaine hydrochloride | marinol |
| marplan | matulane | mavik |
| maxair | maxalt | maxaquin |
| maxidex | maxipime | maxitrol |
| maxzide | mazindol | mazipredone |
| md-76r | md-gastroview | mdp-bracco |
| mebendazole | mecasermin recombinant | mecasermin rinfabate recombinant |
| mechlorethamine hydrochloride | meclizine hydrochloride | meclofenamate |
| meclofenamate sodium | mecloqualone | medetomidine |
| medifoxamine | medipren | medrol |
| medroxyprogesterone acetate | mefloquine | mefloquine hydrochloride |
| mefoxin | megace | megatope |
| megestrol acetate | melperone | melphalan hydrochloride |
| memantine | menest | menopur |
| menostar | menotropins | mentax |
| menthol | meperidine | meperidine hcl |
| meperidine hydrochloride | mephyton | mepivacaine hydrochloride |
| meprobamate | mepron | meptazinol |
| mercaptopurine | meridia | meropenem |
| merrem i.v. | mesalamine | m-eslon |
| mesna | mesnex | mesoridazine |
| mestinon | metadate | metaglip |
| metalyse | metampicillin | metaproterenol |
| metaproterenol sulfate | metaraminol bitartrate | metastron |
| metformin | metformin hydrochloride | methacholine chloride |
| methadone | methadone hydrochloride | methadose |
| methaqualone | methazolamide | methenamine hippurate |
| methergine | methicillin | methimazole |
| methocarbamol | methohexital sodium | methotrexate |
| methotrexate sodium | methoxsalen | methprylon |
| methsuximide | methyclothiazide | methyldopa |
| methyldopate hydrochloride | methylin | methylphenidate |
| methylphenidate hydrochloride | methylprednisolone | methylprednisolone acetate |
| methylprednisolone sodium succinate | methyltestosterone | methyphenidate |
| methyprylon | methysergide | metipranolol |
| metoclopramide | metoclopramide hydrochloride | metofenazate |
| metolazone | metomidate | metopimazine |
| metopirone | metopon | metoprolol |
| metoprolol tartrate | metralindole | metro i.v. |
| metrocream | metrogel | metrolotion |
| metronidazole | metvixia | mevacor |
| mexiletine hydrochloride | mexitil | miacalcin |
| mianserin | micafungin sodium | micardis |
| miconazole | micort-hc | micro |
| micro+4/micro+5/micro+6/micro cr/micro cu/micro i/micro mn/micro se | microderm | microgestin |
| micronase | micronor | microzide |
| midamor | midazolam | midazolam hydrochloride |
| midodrine hydrochloride | mifeprex | migergot |
| migranal | milnacipran | milophene |
| milrinone lactate | miltown | mimpara |
| minaprine | minestrin | minipress |
| minirin | minitran | minizide |
| minocin | minocycline | minocycline hydrochloride |
| minoxidil | mintezol | miochol |
| miostat | miradon | miralax |
| miraluma | mirapex | mirapexin |
| mircette | mirena | mirtazapine |
| misoprostol | mithracin | mitomycin |
| mitoxantrone | mitoxantrone hydrochloride | mivacron |
| mivacurium chloride | mixtard | m-m-rvaxpro |
| moban | mobic | mobicox |
| moclobemide | moderil | modicon 28 |
| moduretic | moexipril hydrochloride | mofegiline |
| molindrone | mometasone furoate | monistat |
| monodox | monoket | monopril |
| monotard | monurol | morphine |
| morphine sulfate | motofen | motrin |
| moxifloxacin hydrochloride | mpi dmsa kidney reagent | mpi dtpa kit - chelate |
| mpi indium dtpa in 111 | ms contin | mucinex |
| mucinex d | mucomyst | multi-11/multi-12 |
| multihance | mupirocin | muse |
| mustargen | mutamycin | myambutol |
| mybanil | mycamine | mycelex |
| mycobutin | mycodone | mycophenolate mofetil hydrochloride |
| mycostatin | mydriacyl | myfortic |
| mykacet | mylaramine | myleran |
| mylotarg | mymethasone | myobloc [botulinum toxin type b] |
| myocet | myoview | myozyme |
| myphetane | myproic acid | mysoline |
| mytelase | mytozytrex | m-zole |
| nabilone | nabumetone | nadolol |
| nadrolone decanoate | nafazair | nafcillin |
| nafcillin sodium | naftin | naglazyme |
| nalbuphine | nalbuphine hydrochloride | nalfon |
| nalidixic acid | nallpen | nalmefene |
| nalmefene hydrochloride | nalorphine | naloxone |
| naloxone hydrochloride | naltrexone | naltrexone hydrochloride |
| namenda | nandrolone decanoate | naphazoline hydrochloride |
| naphcon | naprapacle | naprelan |
| naprosyn | naproxen | naproxen sodium |
| naratriptan | narcan | nardil |
| naropin | nasacort | nasalcrom |
| nasarel | nascobal | nasonex |
| natacyn | natrecor | naturetin-5 |
| navane | navelbine | nebupent |
| nedocromil sodium | nefazodone | nefazodone hydrochloride |
| nefopam | neggram | nelarabine |
| nembutal sodium | neo tect kit | neoclarityn |
| neo-fradin | neomycin | neomycin sulfate |
| neopap | neoral | neorecormon |
| neo-rx | neosar | neospect |
| neosporin | nephramine | nesacaine |
| nesacaine-mpf | nesiritide recombinant | nespo |
| neulasta | neumega [oprelvekin] | neupogen [filgrastim] |
| neupopeg | neupro | neurobloc |
| neurolite | neurontin | neutrexin |
| neutrospec [technetium fanolesomab] | nevanac | nexavar |
| nexium | nexium iv | niacin |
| niacor | niaspan | nicardipine hydrochloride |
| nicergoline | nicoderm cq | nicorette |
| nicotine | nicotine polacrilex | nicotrol |
| nifedipine | nilandron | nilstat |
| nimbex | nimotop | nipent |
| niravam | nisoxetine | nitro-dur |
| nitrofurantoin | nitrofurazone | nitroglycerin |
| nitrol | nitrolingual | nitropress |
| nitrostat, nitroglycerin | nitrous oxide | nix |
| nizatidine | nizoral | nolvadex |
| nomifensine | nonafact | norco |
| nordette-28 | norditropin | norditropin nordiflex |
| norepinephrine bitartrate | norethin | norethindrone |
| norethindrone acetate | norflex | norgesic |
| norinyl | noritate | normosol |
| noroxin | norpace | norplant |
| norpramin | nor-qd | nortrel |
| nortriptyline | nortriptyline hydrochloride | norvasc |
| norvir | novamine | novantrone |
| novo venlafaxine xr | novo-acebutolol | novo-acyclovir |
| novo-betahistine | novo-bicalutamide | novocain |
| novo-fenofibrate-s | novolin | novolog |
| novo-meloxicam | novo-mirtazapine | novomix |
| novonorm | novo-ondansetron | novo-pramipexole |
| novorapid | novo-risperidone | novoseven |
| novo-sumatriptan | novo-tamsulosin | novothyrox |
| noxafil | nozinan | nph iletin i |
| nubain | nulytely | numorphan |
| nutracort | nutrilipid | nutropin |
| nuvaring | nydrazid | nyracta |
| nystatin | nystop | obestatin |
| octocaine | octreoscan | octreotide acetate |
| ocuclear | ocufen | ocuflox |
| ocupress | ocusulf-10 | ofloxacin |
| ogen | ogestrel | olansek |
| olanzapine | olazapine | olopatadine hydrochloride |
| olux | omacor | omeprazole |
| omnicef | omnipaque | omniscan |
| omnitrope | omoconazole | oncaspar [pegaspargase] |
| ondansetron | ondansetron hydrochloride | ondansetron hydrochloride dihydrate |
| ondansetron omega | onsenal | ontak [denileukin diftitox] |
| opatanol | opcon-a | ophthaine |
| ophthetic | opticrom | optimark |
| optipranolol | optiray | optison |
| optisulin | optivar | optruma |
| opulis | orabase | oracort |
| oralone | oramorph | orap |
| orapred | oraqix | orelox |
| oretic | orfadin | orgalutran |
| orphenadrine | orphenadrine citrate | orphengesic |
| ortho cyclen | ortho evra | ortho tri-cyclen |
| ortho-cept | orthoclone [muromonab- cd3] | ortho-est |
| ortho-novum | orudis kt | oruvail |
| orvaten | osigraft | osmitrol |
| osseor | osteogenic protein-1 i | oticair |
| ovcon | ovide | ovidrel |
| ovitrelle | oxacillin | oxacillin sodium |
| oxaliplatin | oxandrin | oxaprozin |
| oxazepam | oxilan | oxistat |
| oxprenolol | oxsoralen | oxybutynin chloride |
| oxybutynin nicobrand | oxycet | oxycodone |
| oxycontin | oxymorphone | oxymorphone hydrochloride |
| oxytocin | oxytrol | pacerone |
| paclitaxel | palonosetron | palonosetron hydrochloride |
| pamelor | pamidronate disodium | pamine |
| pamine forte | pancuronium | pancuronium bromide |
| pandel | panitumumab (vectibix) | panixine disperdose |
| panretin | pantoloc | pantoprazole sodium |
| papaveretum | papaverine | paracaine |
| parafon | paraplatin | parareg |
| parcopa | paremyd | paricalcitol |
| parlodel | parnate | paromomycin sulfate |
| paroxetine | paroxetine hydrochloride | paser |
| paskalium | patanol | patrex |
| paxene | paxil | pbz |
| pce | pedea | pediamycin |
| pediapred | pediatric digoxin | pediazole |
| pediazole, erythromycin ethylsuccinate | pediotic | pegademase bovine |
| peganone | pegasys | pegfilgrastim (neulasta) |
| pegintron | pegvisomant | pemetrexed disodium |
| pemoline | penecort | penfluridol |
| penicillin | penicillin g | penicillin n |
| penicillin o | penicillin s | penicillin v |
| penlac | pennsaid | pentamidine isethionate |
| pentamte | pentasa | pentazocine |
| pentazocine hydrochloride | pentazocine lactate | pentetate calcium trisodium |
| pentetate zinc trisodium | pentobarbital | pentobarbital sodium |
| pentolair | pentostatin | pentoxifylline |
| pentoxil | pepcid | peptides |
| percocet | percodan | perflutren |
| pergolide mesylate | pergolike | pericyazine |
| peridex | periochip | periogard |
| periostat | permapen | permax |
| permethrin | perphenazine | persantine |
| pethidine | pexeva | pfizerpen |
| pharmaseal scrub care) | phenazocine | phencaramkde |
| phendimetrazine tartrate | phenelzine | phenergan |
| phenobarbital | phentermine | phentermine hydrochloride |
| phentolamine | phentolamine mesylate | phentytoin |
| phenyhydrazine | phenytek | phenytoin |
| phenytoin sodium | phisohex | phl-alendronate |
| phl-anagrelide | phl-azithromycin | phl-bicalutamide |
| phl-citalopram | phl-doxycycline | phl-hydrochlorothiazide |
| phl-mirtazapine | phl-ondansetron | phl-risperidone |
| phl-simvastatin | phl-ursodiol c | phoslo |
| phosphocol p32 | | phosphodiesterase-5 inhibitor |
| phospholine iodide | phosphotec | photobarr |
| photofrin | phrenilin | physiolyte |
| physiosol | phytonadione | pilocarpine |
| pilocarpine hydrochloride | pilopine | pimozide |
| pindolol | pipamerone | piperacetazine |
| piperacillin | piperacillin sodium | piperacillin sodium/tazobactam sodium |
| piportil 14 | pipotiazine | pirbuterol acetate |
| pirbuterolnaloxone | piroxicam | pirprofen |
| pitocin | pizotifen | pizotyline |
| plaquenil | plasma-lyte | platinol |
| plavix | plegisol | plenaxis |
| plendil | pletal | plicamycin |
| pms-alendronate-fc | pms-anagrelide | pms-azithromycin |
| pms-bicalutamide | pms-carbamazepine | pms-cilazapril |
| pms-digoxin | pms-doxycycline | pms-famciclovir |
| pms-fluconazole | pms-glimepiride | pms-leflunomide |
| pms-meloxicam | pms-ondansetron | pms-pramipexole |
| pms-risperidone | pms-ursodiol | podofilox |
| polocaine | polyeptides | polymyxin b sulfate |
| polypeptide yy | poly-pred | poly-rx |
| polysporin | polytrim | ponstel |
| porfimer sodium | portia-28 | posaconazole |
| potassium acetate | potassium chloride | povidone iodine, |
| pralidoxime chloride | pramipexole | pramlintide acetate |
| pramosone | prandin | pravachol |
| pravastatin | pravigard pac | prazosin |
| precedex | precose | pred forte |
| pred mild | pred-g | prednisolone |
| prednisolone acetate | prednisolone sodium phosphate | prednisone |
| prednisone intensol | prefest | pregnyl |
| prelone | premarin | premasol |
| premphase 14/14 | premplus | prempro |
| prentoxapylline | pre-op | preotact |
| prepidil | prevacare | prevacid |
| prevalite, | prevenar | previfem |
| prevpac | prexige | prezista |
| prialt | priftin | prilosec |
| primacor | primaquine | primatene |
| primaxin | primidone | primsol |
| principen | prinivil | prinzide |
| pritor | pritorplus | proair |
| proamatine | probalan | pro-banthine |
| probenecid | procainamide hydrochloride | procaine |
| procaine hydrochloride | procalamine | procanbid |
| procardia | procaterol hcl | prochlorperazine |
| prochlorperazine edisylate | prochlorperazine maleate | procomvax |
| procoralan | procrit [epoetin alfa] | proctofoam |
| procyclidine | profen | proferdex |
| progesterone | proglycem | prograf |
| prohance | prohance multipack | proleukin [aldesleukin] |
| prolixin | prolixin decanoate | proloprim |
| promazine | prometax | prometh |
| promethacon | promethazine | promethazine hydrochloride |
| promethegan | prometrium | prompt phenytoin sodium |
| pronestyl | propacetamol | propafenone hydrochloride |
| propanolol | propantheline bromide | proparacaine hydrochloride |
| propecia | propentofylline | propine |
| propofol | propoxyphene | propranolol |
| propranolol hydrochloride | propylthiouracil | proquad |
| proquin xr | proscar | prosol |
| prosom | prostacyclin (treprostinil) | prostascint [capromab pendetide] |
| prostep | prostin | protamine sulfate |
| protaphane | proteins | protelos |
| protonix | protonix iv | protopam chloride |
| protopic | protopy | protriptyline |
| proventil | provera | provigil |
| provocholine | prozac | pseudoephedrine hydrochloride |
| psorcon | pulmicort respules | pulmolite |
| pulmozyme [dornase alfa] | puregon | purinethol |
| pylobactell | pyrazinamide | pyridostigmine bromide |
| pyridoxine hydrochloride | pytest | quadramet |
| quelicin | questran | quetiapine |
| quibron | quinapril hydrochloride | quinaretic |
| quinidine gluconate | quinidine sulfate | quinine |
| quintanrix | quinupristin/dalfopristin | quixidar |
| quixin | qvar | rabies immune globulin (bayrab) |
| radiogardase | ramace | ramipril |
| ran-citalo | ran-citalopram | ran-fentanyl |
| ranibizumab (lucentis) | raniclor | ranitidine |
| ranitidine hydrochloride | ran-pravastatin | ran-risperidone |
| rapamune | rapilysin | raptiva |
| rasagiline | ratio-alendronate | ratio-azithromycin |
| ratio-bicalutamide | ratio-bupropion sr | ratio-fentanyl |
| ratio-fosinopril | ratio-glyburide | ratio-meloxicam |
| ratio-ondansetron | ratio-pentoxifylline | ratio-pramipexole |
| ratio-ramipril | ratio-risperidone | ratio-trazodone |
| rayzon | razadyne | rebetol |
| rebif | reboxetine | refacto |
| refludan | regitine | reglan |
| regonol | regranex | regular iletin ii (pork) |
| relafen | relenza | relpax |
| remacemide | remeron | remicade |
| remifentanil | remifentanil hydrochloride | remodulin |
| remoxipride | renacidin | renagel |
| renamin | renedil | renese |
| reno | renocal | reno-dip |
| renografin | renova | reopro [abciximab] |
| replagal | repronex | requip |
| rescriptor | rescula | resectisol |
| reserpine | resonium calcium | restasis |
| restoril | retavase [reteplase] | retin-a |
| retinol | retisert | retrovir |
| revasc | revatio | revex |
| rev-eyes | revia | revlimid |
| reyataz | r-gene 10 | rhinocort |
| rho(d) immune globulin (bayrho-d) | rhodis | rho-nitro |
| rhotral | rho-triamcinolone | rhotrimine |
| rhovane | ribasphere | ribavirin |
| rid mousse | ridaura | rifadin |
| rifamate | rifampin | rifater |
| rilutek | riluzole | rimactane |
| rimantadine hydrochloride | rimonabant | rimso-50 |
| ringer's | riomet | risedronate |
| risperdal | risperdal consta | risperidone |
| ritalin | ritanserin | ritodrine |
| ritodrine hydrochloride | rituxan [rituximab] | riva-atenolol |
| riva-azithromycin | riva-oxazepam | riva-risperidone |
| riva-zopiclone | rivozepam | rizatriptan |
| robaxin | robinul | rocaltrol |
| rocephin | rocuronium bromide | roferon-a [interferon alfa-2a] |
| rogaine | romazicon | rotarix |
| rotateq | rowasa | roxicet |
| roxicodone | roxilox | roxindole |
| rozerem | rubex | rubidium chloride rb-82 |
| rubramin | rubratope | rythmodan, rythmodan la |
| rythmol | s-adenosylmethionine | saizen |
| salagen | salbutamol sterinebs p.f. | salicylate |
| salmeterol xinafoate | salmetrol | saluron |
| samarium sm 153 lexidronam pentasodium | sanctura | sandimmune |
| sandostatin | sandostatin / sandostatin lar | sandoz acebutolol |
| sandoz anagrelide | sandoz atenolol | sandoz azithromycin |
| sandoz bicalutamide | sandoz bupropion | sandoz ceftriaxone |
| sandoz clonazepam | sandoz cyclosporine | sandoz diltiazem |
| sandoz estradiol derm | sandoz famciclovir | sandoz felodipine |
| sandoz fluoxetine | sandoz gliclazide | sandoz glimepiride |
| sandoz glyburide | sandoz leflunomide | sandoz lovastatin |
| sandoz metformin | sandoz metoprolol | sandoz mirtazapine |
| sandoz nabumetone | sandoz nitrazepam | sandoz ondansetron |
| sandoz paroxetine | sandoz pravastatin | sandoz risperidone |
| sandoz salbutamol | sandoz simvastatin | sandoz sotalol |
| sandoz sumatriptan | sandoz ticlopidine | sandoz topiramate |
| sandoz valproic | sandoz zopiclone | sarafem |
| sativex | savene | scandonest |
| sclerosol | scopolamine | seasonale |
| sebivo | secobarbital sodium | seconal sodium |
| secremax | secretin synthetic human | secretin synthetic porcine |
| sectral | sedapap | selegiline |
| selegiline hydrochloride | selenium sulfide | selepen |
| selsun | semprex-d | sensipar |
| sensorcaine | septra | serevent |
| sermorelin acetate | seromycin | serophene |
| seroquel | serostim | serpalan |
| sertindole | sertraline | sevoflurane |
| shade uvaguard | sibutramine | sifrol |
| sildenafil | silgard | silvadene |
| simulect | simvastatin | sincalide |
| sine-aid | sinemet | sinequan |
| singulair | sinografin | skelaxin |
| skelid | sodium acetate | sodium benzoate/sodium phenylacetate |
| sodium bicarbonatee | sodium butabarbital | sodium chloride |
| sodium chromate | sodium ferric gluconate complex | sodium iodide |
| sodium lactate | sodium nitroprusside | sodium p.a.s. |
| sodium phosphate | sodium polystyrene sulfonate | sodium tetradecyl sulfate |
| solage | solaraze | soltamox |
| solu-cortef | solu-medrol | soma |
| somatropin recombinant | somatuline | somavert |
| sonata | sonazine | sonovue |
| sorbitol | soriatane | sorine |
| sotalol | sotalol hydrochloride | sotradecol |
| sotret | soyacal | spectazole |
| spectinomycin hydrochloride | spectracef | spectrobid |
| spheramine | spiperone | spiriva |
| spironolactone | sporanox | sprintec |
| sprycel | sps: sodium polystyrene sulfonate | ssd |
| ssd: silver sulfadiazine | stadol | stalevo |
| starlix | stelazine | stemetil |
| sterile provocholine solution | sterile vancomycin hydrochloride | steri-stat |
| steroids | stie-cort | stieprox |
| stimate | stocrin | strattera |
| streptase [streptokinase] | streptomycin sulfate | streptozocin |
| striant | strifon | stromectol |
| strontium chloride sr-89 | subtimaze | suboxone |
| subutex | succinylcholine chloride | sucraid |
| sucralfate | sudafed | sufenta |
| sufentanil | sufentanil citrate | sular |
| sulf-10 | sulfacel-15 | sulfacetamide sodium |
| sulfadiazine | sulfamethoprim | sulfamethoxazole/trimethoprim |
| sulfamylon | sulfasalazine | sulfatrim |
| sulfentanil citrate | sulfinpyrazone | sulfisoxazole |
| sulindac | sulpiride | sumatriptan |
| sumatriptan succinate | sumycin | suprane |
| suprax | suprefact | surfactants |
| surgam | surmontil | survanta |
| sustiva | sutent | symbicort turbuhaler |
| symbyax | symlin | symmetrel |
| synacort | synagis | synalar |
| synalgos-dc | synarel | synera |
| synercid | synthroid | syprine |
| tab-profen | tachosil | tacrolimus |
| tagamet | talacen | talc |
| taluvian | talwin | tambocor |
| tamiflu | tamofen | tamoxifen citrate |
| tandospirone | tao | tarceva |
| targretin | tarka | taro-paroxetine |
| tasmar | tavist | taxol |
| taxotere | tazicef | tazorac |
| taztia | technecoll | technelite |
| technescan | technescan gluceptate | technescan maa |
| technescan mag3 | technescan pyp kit | technetium tc 99m |
| technetium tc-99m albumin | technetium tc-99m apcitide | technetium tc-99m bicisate |
| technetium tc-99m depreotide | technetium tc-99m disofenin | technetium tc-99m exametazime |
| technetium tc-99m gluceptate | technetium tc-99m | technetium tc-99m medronate |
| | mebrofenin | |
| technetium tc-99m mertiatide | technetium tc-99m oxidronate | technetium tc-99m pentetate |
| technetium tc-99m pyrophosphate | technetium tc-99m red blood cell | technetium tc-99m sestamibi |
| technetium tc-99m succimer | technetium tc-99m sulfur colloid | technetium tc-99m tetrofosmin |
| teczem | tegretol | teicoplanin |
| telmisartan boehringer ingelheim pharma kg | telzir | temazepam |
| temodal | temodar | temovate |
| tencon | tenecteplase boehringer ingelheim pharma kg | tenex |
| teniposide | tenoretic | tenormin |
| tensilon | tenuate | tequin |
| terazol | terazosin hydrochloride | terbinafine |
| terbutaline | terbutaline sulfate | terconazole |
| terguride | teril | teriparatide recombinant human |
| terra-cortril | terramycin | teslac |
| teslascan | tessalon | testim |
| testosterone | testosterone cypionate | testosterone enanthate |
| testosterone propionate | testosteroneacetate | testosteroneenanthate |
| testosteroneproprionate | testred | tetanus immune globulin (baytet) |
| tetracycline hydrochloride | tetrahydrocannabinol | tetrex |
| teveten | tev-tropin | texacort |
| thalitone | thallous chloride tl-201 | thalomid |
| tham | thelin | theo-24 |
| theochron | theolair | theophylline |
| thermazene | theroxidil | theryttrex |
| thiamine hydrochloride | thioguanine | thioridazine |
| thioridazine hydrochloride | thiotepa | thiothixene |
| thiothixene hydrochloride | thorazine | thyrogen |
| thyrolar | thyrosafe | thyroshield |
| thyrotropin alfa | tiagabine | tianeptine |
| tiazac | ticar | ticarcillin disodium |
| ticarcillin/sulbactam | ticlid | ticlopidine hydrochloride |
| tigan | tigecycline | tikosyn |
| tilade | timentin | timolide |
| timolol | timolol maleate | timoptic |
| tindamax | tinzaparin sodium | tioconazole |
| tiopronin | tiotropium bromide monohydrate | tirofiban hydrochloride |
| tis-u-sol | tizanidine | tizanidine hydrochloride |
| tnkase [tenecteplase] | tobi | tobradex |
| tobramycin | tobramycin sulfate | tobrasone |
| tobrex | tofenacin | tofranil |
| tolazamide | tolbutamide | tolcapone |
| tolectin | tolfenamate | tolfenamicacid |
| tolinase | tolmetin sodium | topamax |
| topicort | topilox | topiramate |
| toposar | topotecan hydrochloride | toprol |
| torecan | torsemide | t-phyl |
| tpn electrolytes | tracleer | tracleer |
| tracrium | tractocile | tramadol |
| trandate | tranexamic acid | tranmep |
| transderm scop | tranxene | tranylcypromine |
| trasylol | travatan | trazec |
| trazodone | trazodone hydrochloride | trecator |
| trelstar | trental | treprostinil sodium |
| tretinoin | trexall | triacelluvax |
| triacet | triacin-c | triamcinolone acetonide |
| triamcinolone hexacetonide | triamterene | triazolam |
| tricor | triderm | tridesilon |
| tridione | triethylperazine | trifluoperazine |
| trifluoperazine hydrochloride | trifluperidol | triflupromazine |
| trifluridine | triglide | trihexyphenidyl |
| trihexyphenidyl hydrochloride | trileptal | tri-luma |
| trilyte | trimeprazine | trimethobenzamide |
| trimethobenzamide hydrochloride | trimethoprim | trimethoprim sulfate |
| trimetrexate glucuronate | trimipramine | trimox |
| trinipatch | tri-norinyl | triostat |
| triphasil | tri-previfem | triprolidine hydrochloride |
| triptorelin pamoate | trisenox | tri-sprintec |
| tritanrix hepb | trivagizole | trivora |
| trizivir | trobicin | tromethamine |
| trophamine | tropicacyl | tropicamide |
| tropisetron | trosec | trudexa |
| truphylline | trusopt | truvada |
| tryptophan | tussigon | tussionex |
| twinject | twinrix | tygacil |
| tylenol | tylox | tysabri |
| tyzine | u-cort | ultane |
| ultiva | ultracet | ultram |
| ultratag | ultratard | ultra-technekow |
| ultravate | ultravist | unasyn |
| uniphyl | uniretic | unisom |
| unithroid | univasc | uprima |
| urea | ureaphil | urecholine |
| urex | urispas | urocit |
| urofollitropin | urokinase | urologic |
| uromax | uroxatral | urso |
| ursodiol | uvadex | vagifem |
| vagistat | valcyte | valdyn |
| valium | valnac | valproate sodium |
| valproic acid | valproicacid | valstar |
| valtrex | valtropin | vancocin hydrochloride |
| vancomycin | vandazole | vaniqa |
| vaniqa | vanos | vantas |
| vantin | vaprisol | vardenafil |
| vaseretic | vasocidin | vasocon |
| vasotec | vasovist | vecuronium bromide |
| veetids | velcade | velivet |
| velosef | velosulin | venlafaxine |
| venofer | ventavis | ventolin |
| venvia | vepesid | verapamil |
| verapamil hydrochloride | verelan | verluma [nofetumomab] |
| verteporfin | vesanoid | vesicare |
| vexol | vfend | viadur |
| viagra | vibisone | vibramycin |
| vibra-tabs | vicodin | vicoprofen |
| vidaza | videx | vigabatrin |
| vigamox | viloxazine | vinblastine sulfate |
| vincristine sulfate | vinorelbine | vinorelbine tartrate |
| vioform hydrocortisone | viracept | viraferon |
| viraferonpeg | viramune | virazole |
| viread | virilon | viroptic |
| visicol | visine | visionblue |
| visipaque | visken | vistaril |
| vistide | visudyne | vitamin a acid |
| vitamin a palmitate | vitamin d | vitamin k1 |
| vitrase | vitrasert | vitravene |
| vivactil | vivanza | vivelle |
| voltaren | voluven | voriconazole |
| vosol | vospire | vumon |
| vytorin | warfarin sodium | welchol |
| wellbutrin | westcort | wilzin |
| wygesic | xagrid | xalatan |
| xanax | xapit | xatral |
| xeloda | xenical | xenon |
| xeristar | xibrom | xifaxan |
| xigris | xolair | xopenex |
| x-trozine | xylocaine | xyrem |
| yasmin | yentreve | yohimbine |
| ytracis | yttriga | zaditor |
| zafirlukast | zalospirone | zanaflex |
| zanamivir | zanosar | zantac |
| zarontin | zaroxolyn | zartra |
| zavesca | zebeta | zeffix |
| zegerid | zelnorm | zemplar |
| zemuron | zenapax | zerene |
| zerit | zestoretic | zestril |
| zetia | zevalin | ziac |
| ziagen | ziconotide | zidovudine |
| zileuton | zimor | zimostatine |
| zimulti | zinacef | zinc chloride |
| zinecard | ziprasidone | ziprasidone mesylate |
| zithromax | zmax | zocor |
| zofran | zoladex | zoledronic acid |
| zolmitriptan | zoloft | zolpidem |
| zometa | zomig | zonalon |
| zonegran | zonisamide | zopiclone |
| zorbtive | zostavax | zosyn |
| zotepine | zovia | zovirax |
| zuclopenthixol | zyban | zyflo |
| zylet | zyloprim | zymar |
| zyprexa | zyrtec | zyvox |

Multiple drugs listed above are currently undergoing research for delivery to the pulmonary tree. The following discussion provides specific examples, but is not intended to be all inclusive of the rapidly advancing field of research regarding pulmonary delivery of pharmaceuticals. The medical port device and delivery method of the present disclosure is intended to deliver any currently existing and future developed drugs that are currently or become approved for pulmonary delivery as they become available for clinical use.

Research has established that peptides, polypeptides, and proteins are an effective way to deliver medications to the rest of the body via the pulmonary route. Additionally many peptides, polypeptides, and proteins also act themselves as therapeutic agents for the treatment of various conditions. For example, multiple proteins are currently undergoing research to alter metabolism. Over 60% of the U.S. population is considered obese. Obestatin, polypeptide YY and leptin are appetite-suppresing hormones. Ghrelin is an appetite boosting hormone. Rimonabant is a new medication which may be a possible new treatment for obesity. Cannabinoid-1 receptor antagonist SR141716A and opioid antagonist LY255582 are other medications that suppress the appetite. Other hormones, including insulin preparations, have been studied, and Exubera has recently become available in a form suitable for inhalation. Calcitonin is inhalable and can treat osteoperosis, hypercalcemia, and Paget's disease. FSH is a hormone that can treat infertility. Growth hormone can treat growth retardation. TSH can treat hypothyrodism, which can cause fatigue and weight gain. Other hormones undergoing research as inhaled forms include somatostatin and parathyroid hormone. LHRH (luteinizing hormone - releasing hormone), including both agonist and antagonist inhalable forms, are being studied for osteoperosis. An inhaled phosphodiesterase-5 inhibitor for erectile dysfunction is also being studied. Vassopressin analogue is used to treat a number of cardiovascular conditions. Immunoglobulins are used to treat infections, and may in the future be customized and delivered to the patient to treat particular diseases or disorders. These all represent promising protein/peptide-based treatments for various diseases and conditions, and, based on preliminary research, the inhalational route may be the only, or most effective means of delivering these drugs.

The disclosed methods of administering drugs also include the delivery of other forms of genetic material (*e.g.*, DNA and RNA) for treating various conditions such as treatment of the lung lining for persons suffering from cystic fibrosis, similar to stem cell treatments for Parkinsons disease (*e.g.*, affecting brain stem), and diabetes (*e.g.*, affecting Islets of Langerhorn). Another drug including genetic material is dornase alpha, marketed under the trademark Pulmozyme™, recombinant DNAse, rhDNase, which is an enzyme used for cystic fibrosis, etc., to reduce the incidence of infection by hydrolyzing DNA in sputum viscoelasticity. An inhalation form of Interleukin I is being studied for asthma. Interferon therapy is undergoing research for multiple sclerosis and Hepatitis B and C. Survivin gene therapy for pulmonary arterial hypertension and hA1PI (human alpha-1 protease inhibitor) or in-situ gene therapy to reduce certain types of emphysema are also being studied. Gene therapy for cancer treatment or prevention is also being studied. Examples include aerosol gene therapy with replacement of p53 genes for lung cancer, and treatment with inhaled cytotoxic drugs (chemotherapy) for lung cancer.

Exemplary proteins for delivery according to the methods of the present disclosure can be found in the database maintained by UniProt Consortium at the following database http://www.pir.uniprot.org/ (January 26, 2006), which is hereby incorporated by reference in its entirety. Exemplary polynucleotides for delivery for gene therapy and/or other treatment applications can be found at the following databases: http://www.ebi.ac.uk/embi/index.html ((January 26, 2006) (RNA/DNA sequences) (the EMBL Nucleotide Sequence Database, also known as EMBL-Bank, is maintained by the European Bioinformatics Institute (EBI) and produced in collaboration by GenBanK (USA) and the DNA Database of Japan (DDJP)) and http://imgt.cines.fr/IMGT GENE-DB/GENElect?livret=0 (January 26, 2006) (Immunoglobulin and T cell receptor genes) (mainained by the International ImMunoGeneTics Information System®), both of which are hereby incorporated by reference in their entireties. Lipids may also be delivered via the pulmonary route via methods of the present disclosure; exemplary lipids can be found at the following database:http://www.lipidmaps.org/data/structure/index.html (January 26, 2006), maintained by the Lipid Metabolites and Pathways Strategy (LIPID MAPS), which is hereby incorporated by reference herein in its entirety.

Inhaled gases are another class of medications that can be delivered via the systems and methods of the present disclosure. Nitrous Oxide is often used as an anaesthetic. Heliox is used in patients undergoing respiratory distress.

Multiple antibiotics are being studied for inhalation. As noted above, tobramycin has been approved for inhalation. Penicillin, quinolones (Cipro), aztreonam, and other antibiotics for pulmonary and systemic infections have been evaluated. Immunoglobins (antibodies) in an inhaled form are also undergoing evaluation in infections and/or inflammatory states. Recombinant human granulocyte colony stimulating factor (GCSF) strengthens the immune system, and an inhaled form is available.

Central nervous system (CNS) applications of inhaled drugs are also being researched. Nicotine is available in several forms but the present application of the medical port and delivery method proposes benefits and alternatives to tobacco addiction without exposure to the carcinogens of the tobacco products. Inhaled drugs that treat migraine headaches and inhaled narcotics, such as morphine, for treatment of acute or chronic pain are also available. Other CNS drugs undergoing research include entonox (inhaled sedative that is a combination of nitrous oxide and oxygen) and inhaled anxiolytics.

Other novel and diverse drugs can also be delivered to the pulmonary tree. Cyclosporin A (organ transplant rejection medicine) has recently been reported to be advantageous in an inhaled form. Alpha-1 antitrypsin enzyme therapy is being studied for treatment of emphysema and cystic fibrosis. Delivery of saltwater solution two times as salty as the Atlantic Ocean has been beneficial in an inhaled form in cystic fibrosis patients. Some other drugs or medications that have been identified as good candidates for use with the disclosed device are inhaled gases and sedatives/anesthetics like nitrous oxide for pulmonary hypertension or for pain. Desflurane and all the "anes" family of anesthetics are also potential candidates. For instance, Corus Pharma of Seattle Washington is currently investigating inhaled lidocaine for alleviating chronic cough for cancer or chronic emphyzema. Other drugs include anxiolytics such as midazolam, marketed under the trademark Versed™ for reducing anxiety (nasal Versed for children or adults is currently available), zolmitriptan, marketed under the trademark Zomig™, and sumatriptan, marketed under the trademark Imitrex™ (which are currently available as nasal sprays for migraines); and antibiotics such as tobramycin solution, which is currently discussed in literature and is already inhalable for cystic fibrosis and bronchial infections, and vancomycin, which is not yet inhaled. Inhaled steroid drugs such as Pulmicort™ are also currently available and are a good candidate for delivery via inhalation.

Drugs that are currently delivered in suppository format and thus rely on mucous membrane absorption represent another class of drugs that may be appropriate for delivery by the presently disclosed system. A non-limiting example of such a suppository-based drug is promethazine, marketed under the trademark Phenergan™, for dizziness and nausea, which is also available orally.

Other pulmonary drugs currently known and that can be used with the disclosed device include, but are not limited to, inhaled prostaglandins such as for newborns to correct patent ductus arteriosis (which closes the bypass hole in the heart); nitrolingual (a nitrogylcerin) pumpspray, which is FDA-approved (lingual spray) for treating coronary artery disease such as angina; and inhaled antihistamines such as azelastine, marketed under the trademark Astelin™, and DDAVP nasal spray, which acts as an antidiuretic by having an effect on the kidneys.

As noted above, some drugs are not currently available for pulmonary administration but are likely candidates for delivery via patient inhalation. These include, for example, inhaled arthritis treatments and vaccines, such as an influenza nasal vaccine (for example that marketed under the trademark Flumist™, which is currently delivered by syringe as a flu vaccine) and TB vaccines.

Drugs for reducing flu symptoms, such as Virazole™, which is available in aerosol form for fighting the effects of Respiratory Syncytial Virus (RSV), are also of particular interest. The presently disclosed systems and methods take advantage of such drugs that are currently available for pulmonary delivery by providing different degrees of dealing with flu virus such as avian flu virus. In the first instance, the disclosed device provides a comfortable, filter system for filtering out pathogens. Secondly, it can be used in conjunction with the medi port of the disclosed device to deliver ribavirin for inhalation, USP, marketed under the trademark Virazole™, or another suitable drug. Thirdly, it can be used in conjunction with devices (such as described in U.S. Patent Application No. 11/412,231, which is hereby incorporated by reference in its entirety) in which ultraviolet light is used to destroy the DNA, RNA, or pathogens that enter the air stream in spite of the filtering system.

The term "pharmaceutical drug" as used herein is also intended to encompass the free acids, free bases, salts, amines, and various hydrate forms including semi-hydrate forms of the drugs mentioned above, as well as pharmaceutically acceptable formulations of such drugs that are formulated in combination with pharmaceutically acceptable excipient materials generally known to those skilled in the art, preferably without other additives such as preservatives. In some embodiments, the drug formulations do not include additional components such as preservatives, which may cause adverse effects. Thus, such formulations consist essentially of a pharmaceutically active drug and a pharmaceutically acceptable carrier (*e.g.*, water and/or ethanol). However, if a drug is liquid without an excipient, the formulation may consist essentially of the drug, which has a sufficiently low viscosity that it can be aerosolized using a respirator device of the present disclosure. In other embodiments, drug formulations may include one or more active ingredients, a pharmaceutically acceptable carrier and/or excipient, as well as other compounds such as, but not limited to, emulsifiers, buffers, preservatives, and the like, as appropriate.

As used herein the term "formulation" generally refers to any mixture, solution, suspension or the like that contains an active ingredient and a carrier and has physical properties such that when the formulation is moved through the respirator device as described herein, the formulation is in a form that is delivered/inhaled/blown by positive pressure into the lungs of a patient. The active ingredient may be any pharmaceutically active drug (as defined above), or diagnostic or imaging agent. The carrier may be any pharmaceutically acceptable flowable agent that is compatible for delivery with the active agent. Useful drugs include drugs defined above, systemically-active drugs delivered to the airways, and useful diagnostics including those used in connection with ventilation imaging. The formulation may also comprise genetic material dispersed or dissolved in a carrier, where the genetic material (when in a cell of the patient) expresses a pharmaceutically active protein or peptide. Formulations may be, for example, solutions, *e.g.*, aqueous solutions, ethanoic solutions, aqueous/ethanoic solutions, saline solutions, colloidal suspensions and microcrystalline suspensions. In embodiments, formulations can be solutions or suspensions of drug in a low boiling point or high vapor pressure propellant. In some embodiments, the formulations can be in solid form. Solid form preparations include powders, tablets, dispersable granules, and capsules. Solid form preparations will be vaporized or aerosolized by the disclosed respirator device, as described hereinafter, so as to be inhaled by a host or patient. Pharmaceutically acceptable excipients can be volatile or nonvolatile. Volatile excipients, when heated, are concurrently volatilized, aerosolized and inhaled with the pharmaceutical drug. Classes of such excipients are known in the art and include, without limitation, gaseous, supercritical fluid, liquid and solids. The following is a list of exemplary carriers within the classes: water; terpenes, such as menthol; alcohols, such as ethanol, propylene glycol, glycerol and other similar alcohols; dimethylformamide; dimethylacetamide; wax; supercritical carbon dioxide; dry ice; and mixtures thereof.

Multiple drugs, drug classes, and evolving therapies (inhaled proteins, genetic material, gases) are being developed to use the inhalation route (nasal, tracheobronchial and alveolar areas). The medical port device disclosed herein and method of delivery is applicable to FDA approved drugs, drugs undergoing current development and any future medications or drugs that can be delivered pulmonically (or via inhalation).

The above drugs and formulations are referenced as being currently or potentially delivered by inhalation or utilized by the respiratory or pulmonary system. It will be appreciated that delivery to nasal passageways and nasal membranes is also within the scope of the present disclosure, and the above drugs and formulations discussed are subject to delivery by the nasal route as well.

While the term medication or drugs is used in the present disclosure, these terms are used widely to include any substance that may have some beneficial or treatment purpose, including amongst other things, substances like water vapor, saline solutions, or compounds used to enhance imaging.

### General Description:

The present disclosure provides systems and methods of delivery of medications to the respiratory system of patients who are capable of unassisted breathing by delivering the medications at a positive pressure relative to atmospheric pressure. In embodiments, the medications are delivered at positive pressure in a stream of air, purified air, or a mixture of gases. In embodiments, the present disclosure provides a system and apparatus for inhaled delivery of medications using purified air at a positive pressure. A device that can deliver the inhaled medications in precise doses and that can deliver medications continuously or in time coordinated response to the respiratory cycles of patients or wearers is also provided. Disclosed herein are devices and systems configured to effortlessly deliver pharmaceutical preparations in purified air to lung air spaces of a patient in a highly efficient, controlled, and targeted manner.

The present disclosure provides a breathing apparatus that serves as a vehicle to administer medication to the user. The present disclosure also provides methods and systems for administering a whole host of drugs via inhalation by a patient, including drugs not previously administered via inhalation.

In embodiments of the system and methods of the present disclosure, the device delivers medications to patients where the patient is capable of breathing without external assistance, and thus invasive breathing assistance or intervention in the recipient's own breathing cycle is not required. This is in contrast to mechanical ventilators, which constitute invasive assisted breathing. As a less extreme example, continuous positive airway pressure (CPAP) machines, designed for treating conditions such as sleep apnea, must intervene to correct the patient's breathing pattern whenever breathing difficulties are experienced, thus also constituting assisted breathing. Another example of assisted breathing includes forms of non-invasive ventilation (NIV) which is used for patients with serious respiratory conditions and those experiencing difficulty breathing without assistance, and is generally used as a last step before intubation. The device of the present disclosure, while not requiring additional respiratory effort on the part of the patient, and while providing some assistance to the user by virtue of positive pressure, does not constitute a device for invasive assisted breathing or intervention into the patient's respiratory cycle.

As used herein, "invasive assisted breathing" refers to breathing assistance requiring intervention in the patient's breathing mechanisms, such as by intubation (for full breathing assistance) or correction of irregular breathing patterns, or for use by patients unable to breath adequately on their own. Although not as invasive as intubation, both CPAP and NIV fall within the class of invasive assisted breathing, as used in the present disclosure. As such, invasive assisted breathing methods and devices typically employ higher pressures than the devices and methods of the present disclosure.

On the other hand, "unassisted breathing" as used herein refers to the ability to breath adequately (*e.g.*, has blood oxygen levels within the normal range) without external assistance such as that provided by one of the above discussed "invasive assisted breathing" methods or devices. In embodiments, the device and methods of the present disclosure are use for patients capable of unassisted breathing. Thus, typically, the pressures employed in the present devices and methods will be lower or otherwise less invasive than those required for devices used for invasive assisted breathing, such as a ventilator or a NIV or CPAP machine. In embodiments, the drug is supplied in air, purified air, or a mixture of gases at a pressure of about 1 cm H₂O to about 30 cm H₂O. Typically, the pressures employed in the device of the present invention are low enough that the patient's own breathing pattern (*e.g.* initiation of inhalation and exhalation) is discernable over the machine supplied pressure.

Although the devices and methods of the present disclosure are for use with spontaneously breathing patients who do not require breathing assistance, in some embodiments the device and methods of the present disclosure can be used in combination with a respirator to deliver medications to a ventilated patient. For instance, the present disclosure also includes the use of respirators described in U.S. Patent Application No. 11/533,529 entitled "Respirators for Delivering Clean Air to an Individual User" (which is hereby incorporated by reference herein) in conjunction with the apparatus disclosed herein. The systems and methods of the present disclosure make full, safe, and efficient use of the highly absorptive linings of the lungs as a way to administer a large host of medications.

The drug delivery methods of the present disclosure can also be implemented using existing breathing systems. A large number of air supply masks ranging from masks covering the mouth and nose, to full face masks, to mouth nozzles as in SCUBA gear already exist could be implemented with the disclosed drug delivery methods in embodiments.

In some embodiments, the supply of pure air can be synthesized (as opposed to filtering environmental air), such as by mixing the gases from reservoirs of liquid oxygen, liquid nitrogen, and liquid carbon dioxide. In particular, an embodiment provides a system includes an air mover, *e.g.*, a pump or blower or a system, that provides air under pressure, as in a SCUBA tank, to generate an air stream of clean air. Numerous active respirators are known, *e.g.*, the Positive Air Pressure Respirator (PAPR), manufactured by 3M; the Continuous Positive Airway Pressure (CPAP) system, manufactured by several medical suppliers such as Puritan Bennet and Respironics, which includes a pressurized mask that typically covers the nose for addressing sleep apnea; fire-fighter type face masks connected to chemical air filtration systems; and face masks connected to compressed air cylinders such as SCUBA gear for underwater diving. As discussed above, in some embodiments the presently disclosed drug delivery apparatus can be implemented using such prior art devices. However, with the exception of highly purified air in a pressurized tank, the existing air supply masks do not typically provide highly purified air, down to 20 nanometers, in combination with ozone removal, which means that in certain environments drug chemistry could be effected by the pollutants in the air. Therefore, in some preferred embodiments the mehtods and systems of the present disclosure use respirators described in U.S. Patent Application No. 11/533,529, incorporated above.

While the elimination of pollutants from the air can itself be considered a benefit to the user from the standpoint that environmental irritants of the lungs and other organs are eliminated, a closer examination of the composition of typical outdoor air, and particularly indoor air, reveals that purified air is particular important for ensuring effective and safe drug delivery via the pulmonary route. The importance of purified air for the systems and methods of the present disclosure arises based on the high concentrations and chemical composition of the particles normally found in environmental air. While particle counts vary widely depending on the particular setting, indoor room air may easily contain greater than 10 billion particles per cubic meter, with many of those particles having diameters down to the 20nm range. Moreover, while there is a tendency to think of these particles as being inert objects, a large percentage of these particles are condensed droplets or micro-crystalline particles of organic and inorganic compounds, including such compounds as aromatic hydrocarbons and carbon particulates.

Predicting the chemical composition of pollutants in room air is further complicated by the presence of ozone. While ozone is a harmful pollutant in it's own right, it is also highly reactive. The reaction of ozone with other organically based pollutants results in numerous derivative compounds which have been studied in some detail for outdoor air (the mechanisms of smog creation, etc.) but are not well documented in current literature and are not widely understood in indoor environments. Other organics are also found in indoor air as a result of outgassing by polymers (carpet, upholstery, etc.) or simply as a result of the use of cleaning compounds. One class of organics that have proven particulary active in forming derivative compounds in air when exposed to ozone are terpenes, which are used in many cleaners and air fresheners and which are responsible for the fresh pine or lemon scent of many cleaning products. Terpenes are sometimes employed as a carrier substance for pharmaceuticals (menthol is an example).

Additionally, at a macro scale in solid, or perhaps liquid form, many of these chemical reactions would proceed relatively slowly. But, as is often demonstrated in high school and college chemistry labs, a high surface area to volume ratio increases the reaction rate between two compounds. With many aerosolized pollutant particles in the 20nm range, the particles have a very large surface area to volume ratio resulting in rapidly occurring reactions.

An area of particular concern regarding the risk of undesirable chemical reactions between therapuetic drugs and environmental contaminants is the pulmonary delivery of proteins and peptides including gene therapy. As described in the review article by F.J. Kelly and I.S. Mudway entitled "Protein Oxidation at the Air-Lung Interface," Amino Acids (2003) (hereby incorporated by reference in its entirety) certain undesirable reactions are known to occur between proteins and reactive oxygen or nitrogen species such as ozone or nitrogen dioxide. As explained in greater detail in the article, reactive oxygen and nitrogen species and their secondary lipid and sugar oxidation products may interact with proteins causing reactions such as oxidation of the polypeptide backbone of the protein, peptide bond cleavage, protein-protein crosslinking, and a range of amino-acid side chain modifications. Both aromatic amino acids (*e.g.*, tyrosine, tryptophan, phenylalanine) and aliphatic amino acids (*e.g.*, arginine, lysine, proline, and histidine) may be targets of reactive oxygen and/or nitrogen species, cysteine and methionine, the two sulphur-containing amino acids, appear especially sensitive to oxidation.

The combination of organic and inorganic pollutants with reactive chemistries, high particle counts, the presence of ozone, and uncertain derivatives as the result of ozone's interaction with other compounds make it difficult to predict air chemistry. Due to the possible formation of numerous compounds that would negatively impact the effectiveness of the drug itself, or perhaps result in the creation of compounds that are detrimental to health, introduction of pharmaceuticals into air that has not been adequately purified greatly increases the likelihood of negative effects. Hence, purified air is preferred for the delivery methods of the present disclosure.

With particle counts in environmental air at times measuring in excess of 10 billion per cubic meter in urban areas and with particle sizes down to 20nm, careful consideration must be given to filtration. The standard for most consumer, occupational, and medical filtration devices is currently HEPA grade filtration (99.97% efficiency at 300nm), which would allow in excess of 10 million particles to pass through for every cubic meter of air that is filtered.

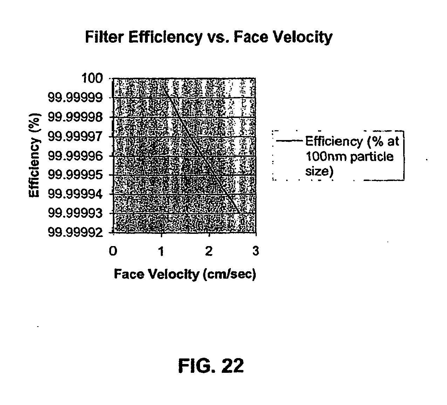

In order to ensure filtration at efficiencies that will eliminate the potential for harmful reactants resulting from high concentrations of unknown airborne chemicals reacting with drugs, both the filter material and overall filter design should be chosen carefully. Filter materials that are capable of these efficiencies *(e.g.,* Lydall Filtration's 6850 grade) are readily available. This technology has been used extensively in settings such as clean rooms, but its use in smaller applications for breathable air such as that described herein is not seen elsewhere in the art. It will be appreciated that, with clean rooms being the principal application for this material and where rapid room air changes are typical, the above, highly efficient filter material is engineered with high flow rates in mind. In such a high flow application, the air passes through the filter material at relatively high velocity. Therefore, the pollutant particles in such an application strike the filter material at a relatively high velocity. The rate of particle penetration depends largely on the kinetic energy of the particle (¹/₂mv²) with particle penetration increasing with velocity. This velocity is termed "face velocity" in the filter industry. The graph in FIG. 22 illustrates the relationship of efficiency to face velocity for a material such as that referenced above.

Based on this information, the goal for maximum filtration efficiency is to utilize the filter materials described above at relatively low face velocities. At a given flow rate, face velocity is inversely proportional to filter area. Thus, the present disclosure uses larger areas than required to satisfy pressure drop requirements in order to establish very low particle velocities, thereby providing the extremely high efficiencies that are important for combining drugs and purified air. At the same time, flow rates equal to or above that of existing devices is achieved.

As indicated above, filter efficiency in this range and with representative glass microfiber technology (*e.g.*, ULPA grade filters such as those from Lydall Filtration/Separation, Inc., Rochester, NH) is achieved when the face velocity drops below 2cm/sec, and full efficiency is realized as it approaches approximately 1 cm/sec. In preferred embodiments of the present disclosure, air flow rates to the user are approximately 320 slm. With indoor and outdoor particle concentrations at times in excess of 10 billion per cubic meter, filter efficiencies should be very high to ensure that unwanted chemical reactions do not occur between particles and drugs. This is particularly important for small particles (*e.g.*, below 100 nm) that have high surface to area ratios. As stated above, the chemical composition of particles will vary greatly as a function of location, weather, etc. Therefore the near elimination of these potential reactants is important in order to have confidence in the drugs (chemicals) ultimately delivered. As also discussed above existing respirators achieve a filtration efficiency of approximately 99.97% at 300 nm. With indoor air particle concentrations of about 10 billion particles per cubic meter and a pulmonary inspiration volume at rest of up to about 5 liters, filtration at about 99.97% means existing respirators allow passage of more than about 15 thousand particles per inspiration of sizes equal to 300 nm in diameter and more than 150 thousand at sizes of about 25 nm and smaller, which provides an environment where unsafe chemical reactants can result from interactions between these high particle concentrations and injected drugs.

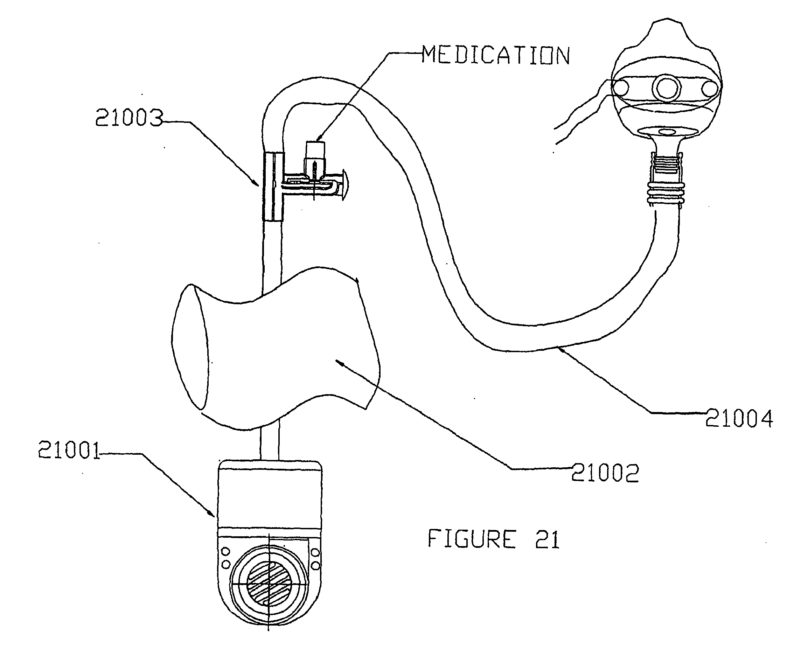

The systems of the present disclosure achieve a high degree of confidence in the chemical composition of delivered medications (*e.g.*, a filtration of about 99.9996%). With the above-described preferred embodiment, the filter area would typically exceed about 500 cm² for this level of filtration. Filter areas of about 2700 cm² up to 5400 cm² in area can be utilized, resulting in filter efficiency of about 99.99996% and about 99.99999% respectively, and corresponding passage of only hundreds of particles per inspiration. In another embodiment, with a flow rate of about 160slm (adequate for the respiratory requirements of an adult at rest), efficiencies of 99.9996% would be realized with filters areas as low as about 250 cm² with maximum efficiencies occurring for areas greater than about 2700 cm². In yet another embodiment (FIG. 21), an air bladder 21002 is employed to hold filtered air in reserve. In this embodiment, large momentary peak inspiration rates (~ 500sim) could be supported with filtration occurring at a much lower average rate. Air supplied to the user via the medical port 21003 and hose 21004 is stored by the blower unit 21001 during exhalation of the user. In this manner, the size requirements of the blower unit are minimized. By maintaining a low average flow rate through the filter, the efficiency is maximized. For instance, at an average flow rate of about 50slm, 99.99999% filtration could be achieved with a filter area of about 830 cm².

Filtration of particulate matter that is present in the air and which forms as a result of reactions between organic particulate matter and ozone a significant improvement; however, ozone, as a molecular level substance, is not removed by simple mechanical filtration and will remain as a pollutant in filtered air. Thus, in some embodiments it is desirable to remove by a reaction or catalytic process in which it is converted to molecular oxygen or into other compounds that are not harmful or that are much less reactive than ozone. One readily available method for reducing or eliminating ozone is the use of an activated carbon filter. This method is achieved through the adsorption of ozone as the air passes over the large surface areas presented by the activated carbon. The activated carbon material may be impregnated into a filter material or alternately, in granulated form, held in place between two layers of filter material. However, the performance of the activated carbon filter deteriorates over time due to the buildup of adsorbed materials and resultant compounds on the surfaces of the carbon. The filter must be continually replaced. Thus, a preferred embodiment includes catalyst that assists in the conversion of ozone ultimately to O₂. MnO₂ (both γ-MnO₂ and β-MnO₂), as well as palladium or palladium oxides, Ag₂O, or other metal oxides such as aluminum oxides and/or copper oxides can be used as a catalyst and can be applied as a coating on surfaces of the delivery device that are in contact with the airstream. The material can also be incorporated into the filter material itself either by impregnation or adhering particles of the catalyst to the filter's fiber matrix. In an exemplary embodiment, the catalyst is incorporated into the chemical makeup of glass fibers of the filter.

Another benefit to the use of a MnO₂ catalyst is that the chemistry involved is also useful for removing SO₂, which is another major air pollutant. Another common pollutant, NO₂, may be catalyzed using different chemistries and with some energy input to drive the reaction. One example is the photocatalysis of oxides of nitrogen when exposed to an irradiated surface of TiO₂. Therefore, additional embodiments of the methods and systems of the present disclosure include using purified air that has also had one or more of ozone, SO₂, and NO₂ effectively removed.

The present disclosure further provides a method and system for supplying the drugs or medication into an air stream, thereby delivering the medication via normal respiration. This is in contrast to albuterol inhalers and other similar devices, which require some extra effort and coordination of the user's inhale cycle with the operation of the device. Typically, drugs are provided to patients in solid, granular, or powder form and are administered as tablets or capsules, or the drug is provided in liquid form and is taken orally (*e.g.*, cough syrup), or is injected into muscle tissue or injected intravenously. Other drugs in turn rely on a delay or slow release mechanism, such as the patch that relies on absorption through the skin. Oral, injection, intravenous, and transdermal delivery methods all have significant drawbacks. Significant hurdles must be overcome for oral delivery of medications due to the requirement that the drug must react correctly to the chemistry of the digestive system. Additionally, once absorbed by the digestive tract, yet another barrier to entering the bloodstream is first pass metabolism in the liver. The obvious drawback to injections and intravenous delivery is the invasive and painful nature of the method, the risk of infection, and the psychological impact of needle insertion. Transdermal delivery, while moderately effective for some readily absorbed drugs like nicotine, is not an efficient means of delivering most drugs.

Pulmonary delivery of drugs avoids all of these issues. Drugs delivered by this route are not subject to complications with digestive tract chemistry and drugs absorbed by the lungs bypass the liver and are therefore not subject to first pass metabolism as are orally delivered drugs. Pulmonary delivery is non-invasive, requiring no needles or surgery. It is well known within the medical field that given the large surface area and sensitive nature of the membranes lining the lungs, that pulmonary delivery is a fast and efficient means of getting medicines into the bloodstream.

Another aspect of the system of the present disclosure is the ability to accurately monitor the pressure and flow parameters of the filtered and medicated air being supplied to the user. Existing devices typically rely on the delivery of either a constant source of medicated aerosol delivered to some vessel or canister through which the user must draw air by his/her own effort or on a system such as an albuterol inhaler, which requires the action of the user for delivery (e.g, the albuterol canister must be depressed in coordination with inhalation). In contrast, embodiments of the present disclosure employ state-of-the art electronic sensors and processors to actively monitor and respond to the respiratory cycle of the user. An array of solid state pressure transducers such as the SM5600 series sensors produced by Silicon Microstructures of Milipitas, CA are used to monitor the pressure conditions within the medical port. Data from the sensors are monitored in real-time by an on-board microprocessor that stores the data collected from the sensors. Through analysis of this data the processor can establish or "learn" baseline respiratory parameters of the user based on approximately one or two minutes worth of data. Once baseline parameters are established the processor may react appropriately to the user's unique requirements and breathing patterns. As one example, the processor may observe pressure readings to detect a particularly rapid or deep (large volume) inhale cycle at its onset. In this manner the processor may cause the port to inject a precisely controlled amount of medicine in the airstream at precisely the correct time for it to be most deeply and effectively inhaled by the user. In another case, the medical port, as controlled by the processor, may administer drugs only during alternate inhalations. The processor may receive input from "smart" drug cartridges in a manner similar to the way ink jet printers for personal computers receive data from ink jet cartridges. This data may be used to instruct the processor regarding the optimal parameters for delivery for the drug and the patient as determined by a doctor of pharmacist. Such data might include information on dosages, proper timing of the dose with the user's respiratory cycle, etc. In one embodiment, the medical port has a data port which may be connected to a device for delivering feedback on the user's condition. As an example, a blood oxygen saturation monitor is used to monitor the user's blood oxygen content and respond appropriately with medications.

Obviously, medicated air could also be delivered in a precisely mixed and continuous fashion if so required. Yet another unique application is for slow and accurate delivery of medicines which are currently delivered as a periodic bolus (such as delivery of albuterol by an inhaler). Slow, gradual delivery of medicines such as albuterol allows patients to receive more appropriate doses without the side effects that come with sudden infusions (such as the "jitters" associated with albuterol inhalers and nebulizers). Existing devices also do not exhibit the ability to deliver inhaled drugs accurately and appropriately for the drug in question and at precise times during the respiratory cycle. The present disclosure provides a method and system for allowing drugs to be administered to the respiratory system of the patient, particularly the lungs, and, furthermore, allows the effectiveness of a drug to be optimized by monitoring the respiratory cycle and controlling the timing by which the medication is administered. By providing the drugs in a purified air stream and in a positive pressure environment, the systems and methods of the present disclosure also make it easier for people with limited respiratory strength and limited coordination, such as children or the elderly, to be effectively medicated.

In addition to removing unwanted pollutants and effectively delivering medications, the present disclosure allows for the temperature and humidity of the air supplied to the user to be controlled so that the most effective conditions for drug delivery and for the comfort of the user are ensured. This is done by the controller using data generated by a temperature and relative humidity sensor such as the HTS2030SMD that is currently available from America Humirel, Inc. in Chandler, AZ. The controller monitors the output of the sensor in order to determine if there is a need to add humidity or remove humidity or raise/lower the temperature of the air stream. The controller can then initiate the appropriate conditioning. Temperature can be raised or lowered using a thermoelectric cooler/heater or an electric resistance heater to modify temperature. It may also initiate the injection of water vapor into the stream to add humidity. Humidity may also be lowered by using an auxiliary condenser or a desiccant as a dehumidifier.

One embodiment makes use of an active type of face mask similar to that described in U.S. Patent Application No. 11/533,529, which is incorporated herein by reference in its entirety, is shown in FIG.s 2A and 2B. The system makes use of an air mover to produce an air stream. As shown in the front view FIG. 2A and side view 2B, the system includes an air supply housing 2400 with a centrifugal blower 2402 covered by a pre-filter 2404. The pre-filter 2404 prevents the blower 2402 from drawing in too many large particles. The air from the blower 2402 is vented radially outwardly and is channeled by the housing wall through the main particle filter 2410, which is mounted above or adjacent to a battery pack 2412. The air is passed out of an outlet port 2420 to which a face mask 2422 is connected by a supply hose 2424. For ease of use, the housing with its blower, filter, and power supply can be attached in "fanny-pack" fashion by means of a belt 2430 to the user. In addition to the above elements the embodiment shown in FIG. 2 includes a medical access port 2440 for introducing a medication 2442, which in this example is an aerosol canister as is commonly used to administer albuterol to asthma sufferers.

The medical access port 2440, which will also be referred to as a medi port 2440. The medi port 2440 comprises a hose adaptor housing 2450 having an air inlet 2452 and an air outlet 2454. In one embodiment, each of the air inlet 2452 and the air outlet 2454 can be provided with a seal arrangement. In one embodiment, the seal is a gasket having three parallel annular ridges to provide more reliable sealing. As shown in this embodiment, the medi port 2440 is connected in the hose 2424. Thus portions of the hose 2424 connect to both the air inlet and the air outlet 2452, 2454. In other embodiments, discussed below, the medi port is connected either at the inlet end or outlet end of the hose 2424. While ease of use may favor the use of a medi port at the inlet end of the hose where the user can readily see what he or she is doing, it is typically preferable, especially in the case of nebulized medicines, to have the medi port as close to the mask as possible. This avoids condensation of medicine along the hose wall and also minimizes any chemical reaction with the pipe material that may cause the pipe to degenerate or cause leaching of undesirable polymers from the pipe into the air stream. In particular, in the embodiment of FIG. 3, two hose adaptors (also referred to as adaptor housings) are shown: one at the downstream end of the hose where it connects to the mask 2422, and one at the upstream end of the hose where it connects to the housing 2400.

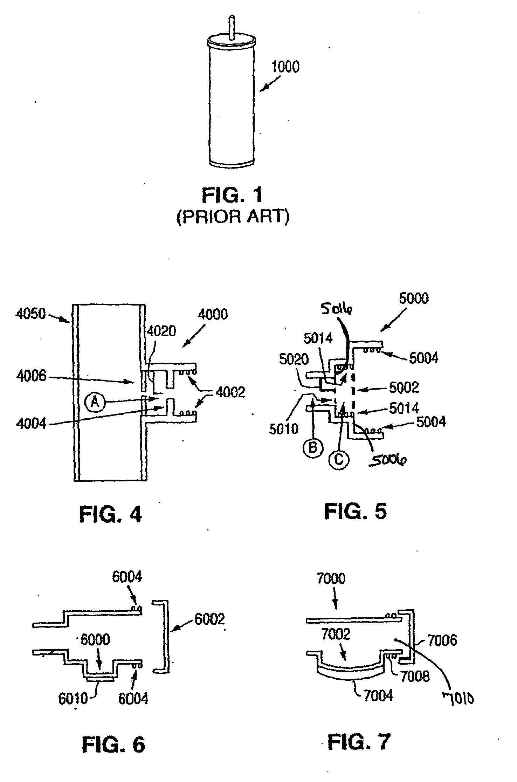

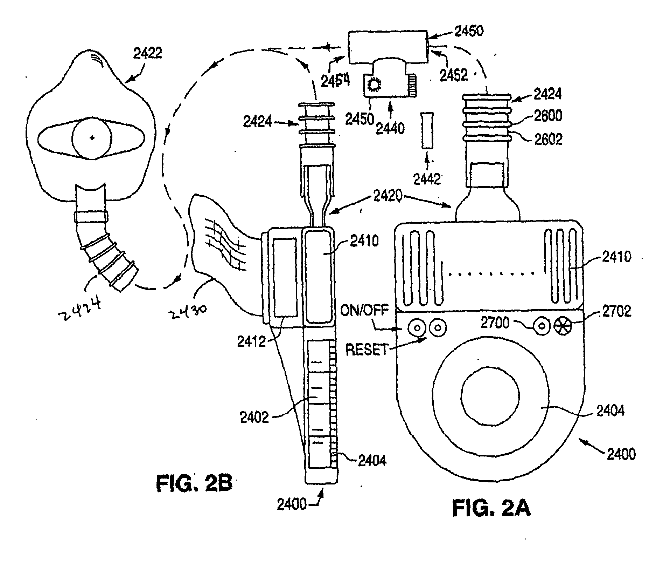

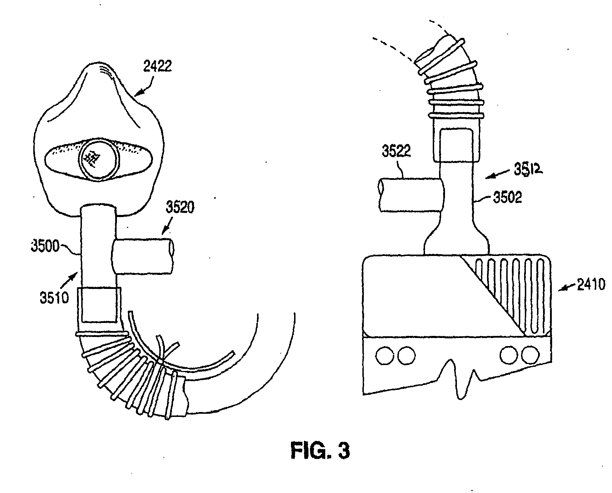

In the embodiment of FIG. 3 the two hose adaptors are indicated by reference numerals 3500 and 3502, respectively. Both medi ports 3510, 3512 also show part of the mixing chamber 3520, 3522. As appears from the FIG. 3 embodiment, the adaptor housings 3500, 3502 and at least part of the mixing chambers 3520, 3522 are connected into the system. When not in use, the unused adaptor housing(s) 3500, 3502 and unused mixing chamber sections 3520, 3522 can be capped by placing a sealing cap over the inlet end(s) of the mixing chamber section(s) 3520, 3522. Such a sealing cap is shown in FIGS. 6 and 7. In one embodiment, the medi ports, such as the medi ports 3510, 3512 are releasably connected to the hose and the mask or air supply housing 2400. To ensure that the medi port is correctly connected, one end may have a female connection and the other end a male connection, as shown in FIG. 3.

As will become clearer from the explanation below, the medi port acts as a vehicle for introducing medication in vaporized or nebulized form into the air stream created by the air mover 2402. This medication is then transported to the user via the hose 2424 or administering the medication to the user. The mask used for this purpose is preferably a fitted mask to allow for precise pressure and flow measurement and therefore dosage control. Also, some embodiments can include a pressure sensor in the mask or hose or elsewhere in the system to detect a loss of positive pressure in the mask and an indicator (visual or audible) of an undesired loss of pressure. In the embodiment of FIG. 2 both a visual alarm 2700 and an audible alarm 2702 are provided on the housing 2400. In fact, such a mask may also be used in contaminated areas even when not used for administering medicines. The system of FIG. 2 also includes an on/off switch for switching the blower 2402 on and off, as well as a reset button for resetting the system once an alarm is triggered. It will be appreciated that during start-up the alarm system is controlled via a time delay to avoid the alarm being triggered, as the system is still in the process of building up the requisite pressure in the mask. Apart from avoiding excessive loss of medication, the use of a fitted mask also provides an extra safeguard (over and above the safeguard provided by a positive pressure in the mask) against ingress of contaminated air into the mask along the mask periphery.

As discussed above, the medi port includes two sections: a hose adaptor and a mixing chamber. FIG. 4 shows one embodiment of a mixing chamber 4000, which is integrally formed with the hose adaptor 4050. The chamber 4000 of this embodiment is provided with an exemplary seal 4002 for better sealingly engaging the outer wall of a canister such as the canister shown in FIG. 1, or a bottle, as is discussed in greater detail below. The chamber 4000 also includes an internal stop or wall 4004 that the front of the canister or bottle abuts once it is pushed into the chamber 4000. Thus it will be appreciated that once the canister or bottle firmly engages the stop or wall 4004, the internal air space 4020 defined by the chamber 4000 is the space between the wall 4004 and an electronically actuated valve 4006. During operation, any vaporized or nebulized medication will therefore fill and be mixed with air in the internal space between the wall 4004 and the valve 4006.

For greater flexibility, embodiments of the presently disclosed device also include an adaptor 5000 for accommodating different size bottles or canisters. In particular, the adaptor 5000 includes a wider input opening for large bottles and canisters. The wider opening includes triple valves 5004 and edge stop 5006 that limits any large bottle from passing the line 5002. The adaptor also includes a second narrower input opening for smaller bottles and canisters, the narrower opening having a seal 5014 for engaging the outer surface of smaller canisters or bottles. In this case the edge stop 5016 stops the bottle or canister at line 5010. It will be appreciated that when the adaptor is used, the adaptor rather than the bottle or canister is slipped into the mixing chamber 4000. Thus when a large bottle is inserted into the adaptor the internal air space is defined by both the mixing chamber space between the wall 4004 and the valve 4006 (depicted by the letter A), as well as the air spaces B and C in FIG. 5. When a smaller bottle or canister is inserted into the adaptor 5000, the cannister or flask fits into the space C, leaving the regions A and B as internal air space for allowing medication to mix with air.

It will be appreciated that other configurations for the mixing chamber and adaptor can be devised without departing from the scope of the present disclosure.

An aerosol is typically provided in the form of a canister such as an albuterol canister, which is typically engaged with the mixing chamber in the manner discussed above. By pressing the canister inward so that its nozzle impinges upon a pin in the chamber such as pin 4020 or a pin in the adaptor, such as pin 5020, a dose of medicine in the form of a puff or bolus is dispensed into the chamber.

Solids in the form of tablets may be placed in the mixing chamber or the adaptor, ane mbodiment of which is shown in FIG. 6. The adaptor of FIG. 6 includes a depression 6000 for receiving the tablet, and an end cap 6002 that engages with double seals 6004 to close the chamber once the tablet has been deposited in the chamber. As shown in FIG. 6, an active vaporizing means in the form of a heating plate 6010 is provided in this embodiment. The plate 6010 may either involve an electric heating element or be implemented as a chemical heating plate that heats when two chemicals react exothermically. In an embodiment that makes use of chemicals it will be appreciated that it is desirable that the chemical remain outside the mixing chamber to avoid any air contamination. Other methods of converting a solid drug into a gaseous form are contemplated to be within the disclosed methods and drug delivery respirator devices. By way of example, one other approach for actively converting a solid into a gaseous form by applying heat is discussed in U.S. Patent No. 7,070,766 to Rabinowitz et al. (incorporated herein by reference), which describes one method of converting a solid to gas whereby a drug, like a migraine or pain relief drug, is coated on a stainless steel leaf with a reactant on the underside that explodes and heats the foil to cause a rapid phase change. The presently disclosed methods include these and other methods of actively vaporizing, e.g, using an energy source such as visible, UV, or IR light, or using an ultrasonic surface with a piezo crystal.

FIG. 7, shows an adaptor 7000 that has a lower depression 7002 with complementary heating pad 7004. An end cap 7006 again engages a double seal 7008. It will be appreciated that the depression serves to retain the liquid over the heating pad while it is being vaporized. In order to administer a liquid into the chamber a pipette or similar dispenser can be used. It will be appreciated that in order to deliver an accurate dose of medication, the amount of liquid dispensed into the chamber has to be accurately measured. In a preferred embodiment, to avoid spillage, a bottle that can deliver an exact amount of liquid is secured to the chamber or an adaptor such as the adaptor shown in FIG. 5, with appropriate accommodation for the nozzle of the bottle. One such bottle that delivers doses to an accuracy of one drop and avoids wastage by ensuring that every drop in a bottle is utilized is the dispensing bottle as described in U.S. Patent No. 6,386,394 to Vollrath et al. (which is incorporated herein by reference). Accurate dosages of medication are then delivered into the chamber by simply charging the bottle and squeezing it. As another form of liquid delivery, especially where the delivery is to be automated by making use of electronic control mechanism, the disclosed device can also employ inkjet printer technology. While FIGS. 6 and 7 show adaptor embodiments for accommodating two different types of medication, it will be appreciated that the changes to the adaptor, such as the depressions 6000, 7002 could also be made in the mixing chamber.

Furthermore, while the embodiment of FIG. 7 is described above for use with liquids, another variation of the embodiment of FIG. 7 is intended for use with tobacco products or nicotine, to smoke in restricted areas or to allow the gaseous medication (in this case tobacco smoke or simply nicotine) to be controlled, thereby allowing the smoker gradually to wean him or herself of the smoking habit. In a preferred embodiment the chemical nicotine is added directly to the air stream in a highly diluted form by the user pushing a wired or wireless button or during a deep inhale cycle as measured by a pressure sensor or continuously. The inlet opening 7010 can be adapted to receive a cigarette, it being appreciated that the mixing chamber will have to be long enough to accommodate the cigarette. Also, a heating pad in such an embodiment is unnecessary. On the other hand, tobacco products or nicotine can be deposited on the concave surface 7002 and heated by means of the heating pad. In all of these uses where a potentially offensive substance is exhaled by the user, a particle filter similar to the filter 2410 can be provided at the air outlet from the face mask. Insofar as a tobacco product that includes harmful products such as tar, is used with the device, the preferred embodiment includes a filter in the adaptor housing, which may be a high quality particle filter to protect not only the user but also to limit particle deposition on the walls of the mask and any air hose used with the device.

One embodiment contemplates a removable, disposable adaptor that is sold with the medication in place, thereby eliminating the need for an inlet opening to the adaptor. Such an embodiment will only provide a single dose per adaptor.

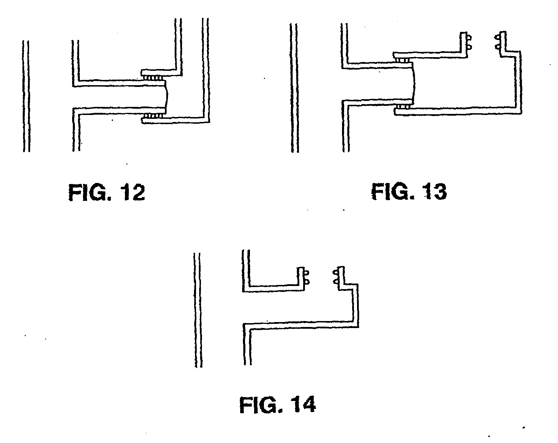

While the above embodiments all show a mixing chamber and a chamber adaptor extending laterally outwardly, the present disclosure is not so limited. One embodiment makes use of a vertically mounted chamber adaptor as shown in FIG. 12. One embodiment makes use of a chamber adaptor with an upwardly facing inlet as shown in FIG. 13. It will be appreciated that instead the mixing chamber itself can have an upwardly facing inlet as shown in FIG. 14. Such embodiments can make it easier to introduce the medication into the chamber with the help of gravity.

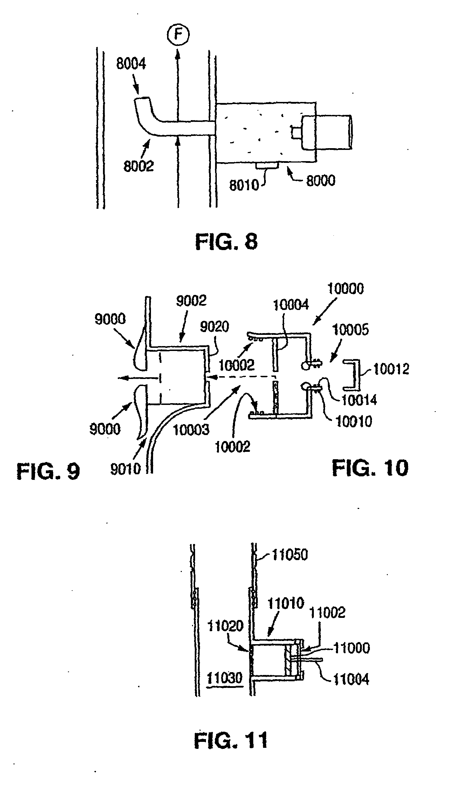

Yet another variation of an adaptor, which is suitable for receiving a bottle or a canister is shown in FIG. 10. In this embodiment the adaptor 10000 has seals 10002 on the inner surface of its outlet end 10003 to engage the outer surface of the mixing chamber 9002 shown in FIG. 9. While the figures depict triple seals, other numbers of seals can be employed. The inlet end 10005 includes outer seals 10010 for engaging with an end cap 10012 when no bottle of canister is present, and has inner seals 10014 for engaging the outer surface of a bottle or canister. The adaptor 10000 of this embodiment includes an end stop or wall 10004 that serves both as abutting surface for the bottle or canister, and also engages the wall 9020 of the mixing chamber. Thus it will be appreciated that the internal air space in this embodiment is defined only by the chamber 9002 and not by the adaptor.

As discussed above, in the case of a liquid or solid medication that is neither in nebulized form nor in aerosol form, a vaporization step has to take place. The vaporizing can be achieved by providing energy to the medication, such as by actively heating the medication. Instead of heat, other forms of energy can be provided to the medication to vaporize it. For instance, physical shaking or the use of ultrasonic agitation can be used as by the agitator 8010 shown in FIG. 8.

Instead, the medication may be of such a nature that it readily vaporizes without external intervention, *e.g.*, passive vaporization.

The above discussion has focused on dispensing the medication into the mixing chamber in aerosol or nebulized form suitable for transportation in an air stream or alternatively dispensing in a form that requires subsequent vaporization. Another important aspect involves the introduction of the aerosol, nebulized, or vaporized medication into the air stream. This involves transferring it in a controlled manner from the mixing chamber into the adaptor housing 2450, 3500, 3502, 4050.

Any suitable method of moving the medication from the mixing chamber into the air stream of the hose adaptor can be used. In one embodiment, the vaporized, nebulized, or aerosol in the mixing chamber 8000 is drawn out by creating a Venturi effect by means of a curved pipe 8002 as shown in FIG. 8. Air flow bends around the pipe 8002 and therefore speeds up to form a low pressure zone at the opening 8004 of the pipe. This draws the material out of the chamber 8000.

Another embodiment making use of the Venturi effect to pull or draw the material from the chamber is shown in FIG. 9. Here baffles 9000 that have a teardrop or aerofoil shape in this embodiment are formed at the outlet to the chamber 9002. An inlet opening or channel is provided to the medical port to serve as the air intake for fresh air entering the mixing chamber.

Instead of or in addition to a Venturi device to suck out the material from the chamber, an air stream can be directed into the chamber to push the material out. The embodiment shown in FIG. 9, in fact, includes such a pushing action as well, as defined by the inlet channel 9010 at the lower end of the lower baffle 9000.

In yet another embodiment the mixing chamber is pressurized *e.g.*, by an external source of a pipe leading to the chamber from a higher-pressure region in the system. This increased air pressure in the chamber serves to push the medicated air out of the chamber whenever the valve between the chamber and the hose adaptor is open.

While the above embodiments have relied on low pressure or an air stream to move the material out of the chamber and into the hose adaptor, another embodiment makes use of a physical propulsion mechanism in the form of a piston 11000, as shown in FIG. 11. The piston may be propelled manually by the user or may be coupled to a motor or spring mechanism to gradually move the piston inward until all of the medicated air in the chamber has been expelled from the chamber. In this embodiment a helical spring 11002 and a rod 11004, for pulling the piston 11000 back to allow it to compress the spring are provided. Once the rod 11004 is released, the tension in the spring 11002 moves the piston into the chamber 11010, expelling the medication filled air through the electronic valve 11020 into the hose adaptor 11030.

FIG.s 12 and 13 show different embodiments of adaptors, while FIG. 14 shows an embodiment of a mixing chamber that all provide for vertical mounting of a bottle to facilitate gravity feed.

In order to control expulsion of air from the mixing chamber into the hose adaptor, a valve mechanism is provided such as the electronic valve 4006 in figure 4, and the valve 11020 in FIG. 11. In the case of electronically actuated valve 4006, an electronic valve as known in the art is used. In the case of valve 11020, an electromechanical shutter mechanism like that found in a camera, is used. In order to control the flow of air through the valve or shutter, the opening or aperture can be controlled. Alternatively, instead of always keeping the opening or aperture open and simply varying the size of the opening, the valve or shutter can be intermittently closed and opened to release small quantities of medication into the air flow.

The controlled manner in one embodiment includes releasing some of the medication every time the user inhales. In one embodiment, the controller monitors the inhalation and exhalation and releases medication according to a certain series, *e.g.* every second or third inhalation, or two inhalations in a row followed by three inhalations where no medication is dispensed. The pattern or series may be changed depending on the nature of the medication. In addition, air pressure or air flow may be taken into account to vary the size of the aperture or the amount of time that it is open, depending on how deeply the person is breathing in. Also, in one embodiment, a button, momentary switch, or some other device for signaling the controller is employed to indicate the user's wish that medication be delivered upon some future event, such as the next inhalation cycle. In this manner the drug could be delivered periodically as preferred by the patient while the benefit of timed delivery is preserved. In another embodiment, the medication can be provided in a continuous manner, rather than in pulses.

As discussed above, the system will include sensors for indicating the rate of flow of air to the user, the output from which will be used by a controller to calculate dosing parameters. The flow in this application may be measured by a number of methods. It may be measured directly by means of a hot wire anemometer, mechanical anemometer, or mass air flow sensor placed in contact with the air stream flowing through the port. Preferably, flow sensing would be performed indirectly using pressure sensors. These sensors can be used with a pitot tube, or some number of sensor, (*e.g.*, three) are placed with access to the air stream on each side of the venturi structure within the port. The controller, based on pressure as measured by the sensors, can then monitor the pressure differential across the venturi and calculate flow based on this information. Use of multiple sensors would allow the controller to average the data, and occasional erroneous readings from individual sensors due to turbulence, etc. could be omitted in order to yield an accurate set of data upon which to base the control of the port functions. In addition, if at least one pressure sensor is included to measure atmospheric pressure, the controller will also be able to monitor the pressure within the medical port, hose, and mask in order to determine if the wearer's respiration creates a negative pressure, indicating inadequate performance of the blower unit. In one embodiment, the controller that controls air flow rate or pressure by controlling power to the air mover may include an algorithm for controlling the shutter or valve to release medication in a controlled manner.

The pressure sensors or flow sensor may be mounted in the adaptor housing and any holes in the adaptor housing or tube for passing wires out of the housing are sealed. This may be done by potting the adaptor housing. In one embodiment, all the sensors and monitors in the adaptor housing are mounted on a printed circuit board that snaps onto an inner surface of the housing by means of clips. To avoid the electronics being exposed to the air stream, a conformal coating is provided over the circuit board with its components. While the controller can also be mounted on the circuit board, the sensors and monitors in another embodiment are connected to a monitor on an external circuit card, or in the air mover housing. In an embodiment where insulin is being administered to the lungs, the device of the present disclosure provides a feedback loop from an insulin monitor to the controller to automatically calculate the requisite amount of insulin to administer based on the detected blood/sugar levels in the user's blood.

In the embodiment where the controller is mounted on the circuit board, wires out of the medi port can be eliminated altogether by providing a separate power supply on the circuit board, *e.g.*, by way of a watch battery.

Power supply to the medical port can also be provided by energy sources such as solar cells, small wind turbines, or fuel cells for use in areas where access to an electric grid is not possible or convenient.

In order to ensure accurate amounts of medication are delivered to the user, it is important to control the amount of drug or chemical introduced into the mixing chamber and the rate of air flow out of the port (into the air stream). If both of these values are known, then the mixing rate and delivery rate may be determined and controlled. The system may deliver a fixed amount of drug to the mixing chamber and then allow this mixture to be drawn from the chamber at the appropriate moments and over the appropriate amount of time, or it may deliver drugs to the mixing chamber as a continuous process.

Once the medication in the chamber is transferred into the air stream it is carried by the hose 2424 (FIG. 2) or the hose 11050 (FIG. 11) to the mask, such as the mask 2422 of FIG. 2.

In embodiments the hose includes an inner lining, the hose is made of a material that does not leach polymers into the air stream, as may otherwise occur, especially with certain kinds of medicines. Furthermore, in embodiments the hose is made from a material or lined with a material that prevents or reduces chemical degradation from exposure to the drug. In yet another embodiment, the hose is releasably connected to allow it to be replaced from time to time. This allows the issue of degradation and drug residue accumulation on the hose inner surface to be addressed.

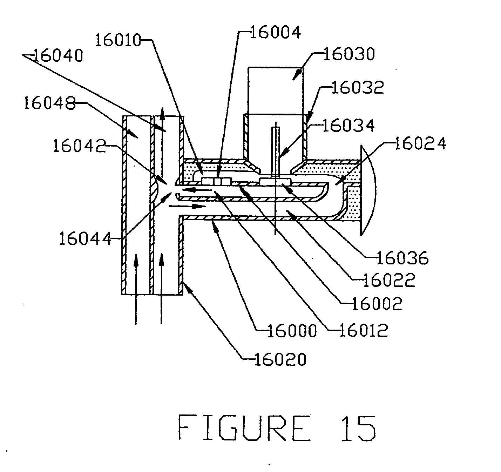

While the above discussed embodiments have made use of a shutter or an electronically controlled valve between the mixing chamber and the adaptor housing, another embodiment provides the shutter or valve to be mounted in the mixing chamber. Such an embodiment is shown in FIG. 15, which includes a mixing chamber 16000 that is divided into two sections 16010, 16012 by a printed circuit board (PCB) 16002. The PCB 16002 provides two air flow paths: one between the upper section 16010 and the lower section 16012 by virtue of a shutter or valve 16004, and one for channeling air flow from the adaptor housing 16020 via a channel 16022 to the upper section 16010. The latter air flow path simply comprises a hole or spacer 16024 in the PCB 16002. A Alternatively, the valve 16004 could be located at the inlet hole from the lower housing to the upper housing to control the inlet 16024 to the mixing chamber rather than the outlet of the mixing chamber. A bottle or canister 16030 is seated in the vertically extending support 16032. In one embodiment, the vertically extending support 16032 can be of a smaller configuration, as for a child-sized mask, such that an larger- *e.g.*, adult-sized canister 16030 cannot fit in the smaller support 16030. In this manner, overmedication of a child or smaller patient can be avoided.

In the case of a canister, a pin 16034 impinges on the nozzle to allow a bolus of medication to be expelled into the upper section 16010. In the case of a liquid dispensed from a bottle or other liquid dispenser, a heating pad or piezo plate 13036 vaporizes the liquid. The air pressure in the upper section 16010 created by the air entering through the hole 16024 forces the air into the lower section 16012 whenever the valve 16004 opens.

The medication is drawn into the channel 16040 of the adaptor housing 16020 by virtue of a Venturi effect created by a curved surfaces 16042, 16044 at the inlet to the adaptor housing 16020. In this embodiment, the adaptor housing 16020 is bifurcated into a medication carrying channel 16040 and a non-medicated air stream channel 16048 to allow air to bypass the Venturi region 16042, 16044 and not force medicated air upon the user.

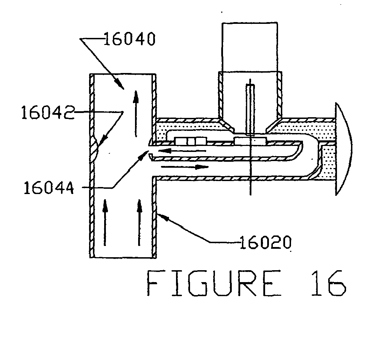

In one embodiment, illustrated in FIG. 16, the medi port, the adaptor housing 16020 is not bifurcated, and includes only one channel 16040. Thus, the medicated air and non-medicated air mix as they bypass the Venturi region 16042, 16044.

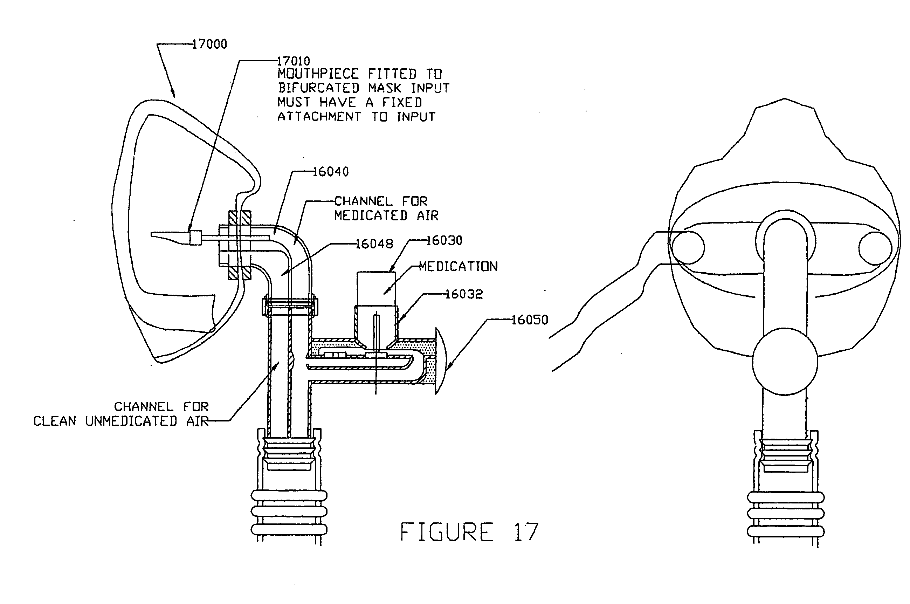

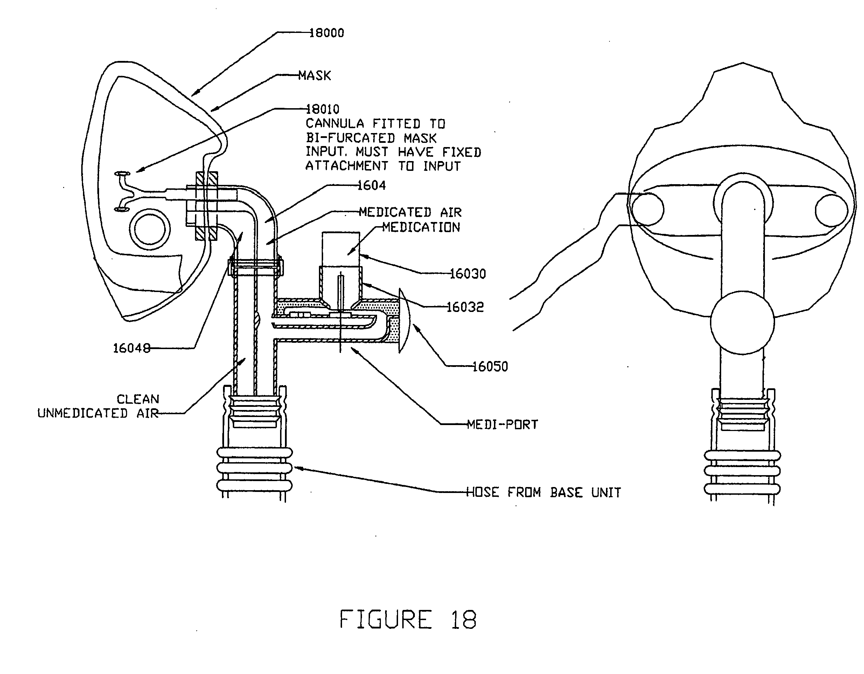

This bifurcated adaptor housing is further illustrated with respect to the embodiments illustrated in FIGS. 17 and 18. FIGS. 17 and 18 show the bifurcated channels 16040, 16048 extending to a face mask 17000, 18000. In the case of face mask 1700, the medication carrying channel 16040 extends to a mouth piece 17010, which in this embodiment is fixedly attached to the mask to avoid inadvertent swallowing or choking hazard. In other embodiments, the mouthpiece or the cannula is releasably attached to allow it to be disposed of after a certain amount of use and replaced with a new mouthpiece or cannula. The addition of a mouthpiece ensures that all of the medicated air reaches the mouth of the user, leading to less medication wastage and more accurate dosage. It will be appreciated that this embodiment is suitable for applications where the medication is preferably inhaled through the mouth. In the embodiment of FIG. 18, the channel 16040 extends to a nosepiece in the form of a cannula 18010. The cannula may be designed to fit into a single nostril allowing the user to alternate delivery between nostrils, or to both nostrils at the same time. This embodiment is preferable for medications that are to be inhaled through the nose, and again provides for more accurate dosage and better delivery than simply filling the mask. In yet another embodiment, where the issue of nose or mouth inhalation is not important, the mouthpiece 17010 and cannula 18010 need not be included. Instead the medication is simply delivered to the mask. Preferably, the mask fits well to minimize loss of medication through the sides of the mask between the user's face and the mask periphery. In order to eliminate any waste products from the medication, the medi port is provided with an end cap 16050 to provide easy access to the interior of the medi port.

As discussed above, the dispensing of the medication into the mixing chamber or the delivery into the air stream may be controlled by a controller on a circuit board in the medi port or by a controller mounted in the blower housing. In embodiments, the drug container has a memory stick attached and may be preprogrammed, *e.g.*, at the factory, to a predefined set of parameters, or by a pharmacy to suit the particular drug, drug concentration, type of dispensing device, age of user or dosage, and any other relevant parameter to dispense according to the particular usage. Programming can be achieved by making use of a wireless interface, *e.g.*, Bluetooth, Zigbee, etc. It will be appreciated that the controller will also gather real time data such as differential pressure, flow rate, inhalation volume of air over time, etc. The controller can utilize this data to adjust drug delivery at the mediport to maintain desired dosage levels. Communication from a controller mounted in the blower housing to the mediport may be via a wire or wireless.

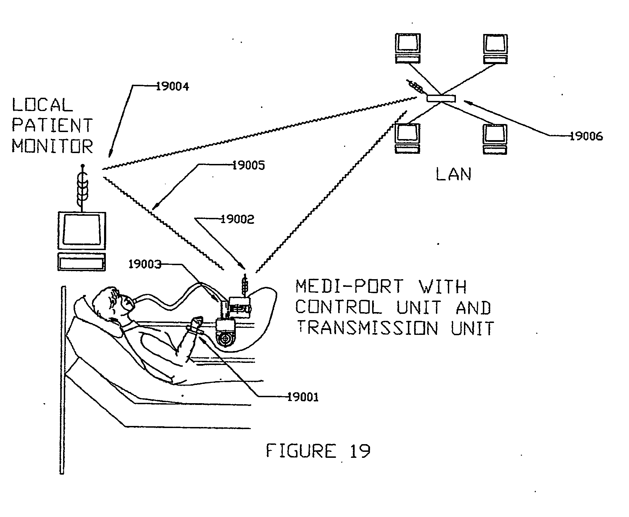

In addition, as illustrated in FIG. 19, the controller, either in the medical port or the blower, may take inputs from blood pressure, heart rate, blood oxygen saturation, or blood glucose sensors 19001, etc. (either wired or wireless) to initiate or stop the dosage of drugs or change the dosage level or frequency based on pre-determined algorithms. The medical port 19003 itself may provide data via a wire, or through a wireless transmitter 19002 to other devices in proximity to the medical port. In this manner, data including, but not limited to, blood pressure, blood oxygen saturation levels, heart rate, blood glucose levels, respiration rates, respiratory volume, etc. can be monitored in real-time, such as on a local computer monitor 19004, which is in communication 19005 with these devices and the medical port 19003. The local monitor 19004, in addition to communicating with the sensors and medical port, may be connected by wire or wirelessly to a network, such as a local area network or wireless router 19006. In a similar manner, the sensors and medical port can be connected by wire or wirelessly to the same local area network or router as the local machine so that all data is available to both the local machine and the network. In this way it is possible for a health care professional such as a nurse or physician to both monitor the condition of the patient remotely and cause the medical port to change dosage, frequency of delivery, temperature, humidity, etc. of the air flow to the patient from a remote location while monitoring the patient in real-time. It will be appreciated that the patient need not be in a hospital setting for this embodiment to be realized and that this capability would work well in a home health care setting. As in the above discussion, the wireless interface protocol could be Bluetooth, Zigbee, or one of the 802.11 standards and wired connections could be serial such as I²C or simple RS232.

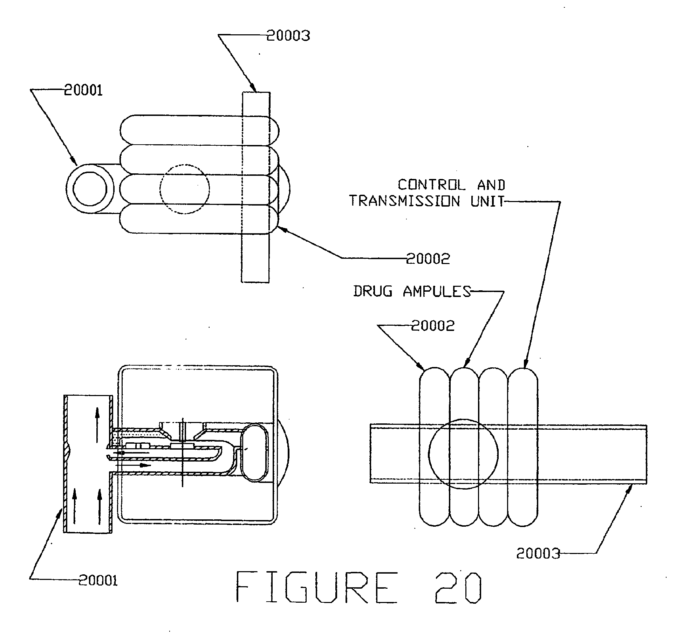

In the embodiment shown in FIG. 20, the mediport 20001 may be fitted with multiple ampules 20002 capable of dosing multiple drugs simultaneously or at different frequencies such as during different or alternating inhalation cycles. In this embodiment the ampules are mounted onto a slide mechanism 20003 and may index into position over the inlet to the medical port, allowing the controller to control which drugs are dispensed. However, the system of Fig 20 need not be the only embodiment for dosing multiple drugs. For instance the medical port of Fig 16 could simply be designed so that there are two or more mixing chambers diametrically opposed to one another, allowing dosing from multiple mixing chambers into a single air stream.

In addition, because in a preferred embodiment, the device can measure the depth and volume of each inhalation cycle, drug delivery can be triggered to occur only in inhalation cycles with a high volume and that are optimal for drug delivery. This is done by continuously measuring the recent history of inhalation cycles for a specific user over the period of several minutes and then comparing the slope and depth (prior to reaching the deepest level of the cycle) of the inhalation curve to trigger drug release during an inhalation. Multiple input measurements may be utilized to confirm certain conditions such as a sudden decrease in cardiac output which would trigger the release of specific drugs and/or, in another embodiment described elsewhere in this application, increase oxygen levels in the inhaled air.

While the above embodiments all make use of a hose to transfer the medication to the face mask, the present disclosure is not so limited. In one embodiment, for example, the medi port is connected directly between a face mask and an air mover housing without any hose being used. Typically the medi port in such a configuration will define an adaptor housing for receiving the outlet from the mixing chamber, and for connecting between the mask and the air mover housing.

Once the medication reaches the mask, the user simply inhales the medication. By providing the ability to deliver only small quantities of medication over a period of time, absorption of the medication is improved. As discussed above, the mask is preferably a fitted mask to minimize the escape of air along the periphery of the mask. One embodiment makes use of a split manifold for supplying air to both the mouth and nose regions of the user. In one such embodiment, a slider is included to physically vary the ratio of air to the nose relative to the air to the mouth. In another embodiment, instead of a mask that covers both mouth and nose, a partial mask for only the nose or only the mouth may be used.

It is anticipated that protection against the delivery of the incorrect drug or incorrect dosage will be incorporated in this system for use with some drugs. These drug and user identification systems may involve simple color coding of medicine containers or geometry constrictions that prevent adult dosages of medicines from being administered from mask systems that fit children. More sophisticated systems may package medicines in containers incorporating bar code or RFID (radio frequency identification) tags that can be checked by the microprocessor in the mask system to confirm the correct drug and correct dosage. In this system, prescriptions may be downloaded to the mask microprocessor, perhaps by an RF protocol such as Bluetooth or Zigbee or by another RFID tag. Such prescriptions inform the mask system of the drug and dosage for the person using the mask. Advanced versions of the system may even confirm the identity of the mask user by their own RF tag or a password. Similarly, statistics of mask use, including user, time and date of use and system condition to confirm correct delivery of medications. This may be especially be done in situations where the recipient of the drug may need to be monitored due to poor memory, attention or because treatment is subject to substance addiction.

It is also anticipated that it may be desirable to prevent small quantities of certain drugs from reaching room air and other non-medicated occupants via being in exhaust air from person's lungs. For example, if a person is using the mask system for providing low dosages of nicotine it is desirable that this potentially addictive substance is not inhaled by other room occupants, even in low doses. This is accomplished by filtering air exiting the mask through filters capable of removing small particles, or even in some cases of chemically deactivating the drub by materials such as activated carbon. In addition, it should be known that the particle filter mentioned above, in a preferred embodiment would be a sterilization chamber fabricated from materials such that the interior surfaces have a high reflectivity in about the 250 nm to 280 nm wavelength range. The sterilization chamber utilizes ultraviolet light generated by mercury vapor lamp(s), light emitting diodes, or other light emitting opto-electronic devices (all such devices emitting UV radiation between about 250 nm and 280 nm) to destroy the RNA or DNA of any airborn pathogens exhaled by the user.

For added comfort, a highly flexible mask is contemplated having a central more rigid portion to define an air space in front of the user's mouth and nose, or that gradually becomes more inflexible toward the mouth and nose region and is most flexible along the periphery. The mask also includes multiple parallel extending seals along the periphery of the mask to provide a better seal to the user's face. In highly critical applications, where any contamination from the outside is to be avoided and reliance on the positive pressure in the mask and the multiple seals is not enough, it is proposed to secure the mask to the user's face by means of an adhesive which makes removal of the mask more difficult and may even require a solvent.

Additionally, to increase compliance for pediatric patients, some embodiments can employ masks molded and decorated to resemble cartoon characters or animals that would entertain children and increase their emotional comfort level with the device. Similarly, the mask can be made in a variety of colors that would be more appealing to both pediatric and adult users. In a similar manner, a communications system using a microphone and speaker system employing a sound processor could be added to facilitate communication through the mask, or, again, to increase compliance for children and perhaps adults by adding fun features (voice harmonization, simulation of cartoon or TV characters, *e.g.*, Darth Vader™, Spongebob Squarepants™, etc.).

While embodiments of the systems and methods of the present disclosure have been described above with respect to a delivery system employing a mask for delivery of the medication and purified air stream, it will be appreciated by those of skill in the art that the methods and systems of the present disclsoure can also be employed for the treatment of intubated patients. The devices and systems described above can be modified as appropriate for use with venitlators and/or respirators adapted for use with intubated patients, as would be appreciated by one of skill in the art.

The present disclosure thus provides for a way of safely administering medication via inhalation of purified air by a patient over time in an actively and precisely controlled manner. While a number of embodiments were discussed above, it will be appreciated that the present disclosure is not limited to these embodiments but could be implemented in other ways without departing from the scope of the present disclosure.

## Claims

1. A method of administering a drug to the respiratory system of a patient comprising:
delivering the drug to the patient using air supplied at a positive pressure relative to atmospheric pressure, wherein the patient is capable of unassisted breathing.

2. The method of claim 1, wherein the air is purified air.

3. The method of claim 1, wherein the air is supplied at a pressure from about 1 cm H₂O to about 30 cm H₂O.

4. The method of claim 1, wherein the drug is delivered to the patient via a mask, wherein the mask is substantially sealed around the patient's nose and mouth.

5. The method of claim 1, wherein the drug is a pulmonary drug.

6. The method of claim 1, wherein the drug is a systemic drug.

7. The method of claim 1, wherein the drug is selected from the group of drugs consisting of: albuterol, albuterol sulfate, atropine sulfate, beclomethasone dipropionate, bitolterol mesylate, budesonide, formoterol fumarate, cromolyn sodium, desflurane, dexamethasone sodium phosphate, dornase alfa, enflurane, epinephrine, ergotamine tartrate, flunisolide, fluticasone propionate, fomoterol fumarate, halothane, iloprost, insulin, ipratropium bromide, isoetharine hydrochloride, isoflurane, isoproterenol hydrochloride, levalbuterol hydrochloride, metaproterenol sulfate, methacholine chloride, mometasone furoate, nedocromil sodium, nicotine, nitric oxide, pentamidine isethionate, pentetate calcium trisodium, pentetate zinc trisodium, pirbuterol acetate, ribavirin, salmeterol xinafoate, sevoflurane, tetrahydrocannabinol, tiotropium bromide monohydrate, tobramycin, trimcinolone acetonide, zanamivir, and combinations thereof.

8. The method of claim 1, wherein the drug is selected from the group of drugs consisting of: 13-cis-retinoic acid, 2-pentenylpenicillin, L-alphaacetylmethadol, S-adenosylmethionine, acebutolol, aceclofenac, acetaminophen, acetaphenazine, acetophenazine, ademetionine, adinazolam, adrafinil, ahnotriptan, albuterol, albuterol, albuterol sulfate, alfentanil, alfentanil HCI, alizapride, allylprodine, alminoprofen, almotriptan, alperopride, alphaprodine, alpidem, alseroxlon, amantadine, ambrisentan, amesergide, amfenac, aminopropylon, amiodarone HCI, amisulpride, amitriptyline, amixetrine, amlodipine, amoxapine, amoxicillin, amperozide, amphenidone, amphetamine, ampicillin, amylpenicillin, andropinirole, anileridine, apazone, apomorphine, apomorphinediacetate, atenolol, atropine sulfate, azacyclonol, azasetron, azatadine, azidocillin, bacille Calmette-Guerin, baclofen, beclomethasone dipropionate, benactyzine, benmoxine, benoxaprofen, benperidol, benserazide, benzpiperylon, benzquinamide, benztropine, benzydramine, benzylmorphine, benzylpenicillin, bezitramide, binedaline, biperiden, bitolterol, bitolterol mesylate, brofaromine, bromfenac, bromisovalum, bromocriptine, bromopride, bromperidol, brompheniramine, brucine, buclizine, budesonide, budesonide; formoterol fumarate, budipine, bufexamac, buprenorphine, bupropion, buramate, buspirone, butaclamol, butaperazine, butorphanol, butriptyline, cabergoline, caffeine, calcium-N-carboamoylaspartate, cannabinoids, captodiamine, capuride, carbamazepine, carbcloral, carbenicillin, carbidopa, carbiphene, carbromal, carfecillin, carindacillin, caroxazone, carphenazine, carpipramine, carprofen, cefazolin, cefinetazole, cefmetazole, cefoxitin, cephacetrile, cephalexin, cephaloglycin, cephaloridine, cephalosporin C, cephalosporins, cephalotin, cephamycin A, cephamycin B, cephamycin C, cephamycins, cepharin, cephradine, cericlamine, cetrizine, chloralbetaine, chlordiazepoxide, chlorobutinpenicillin, chlorpheniramine, chlorpromazine, chlorprothixene, choline, cialis, cilazaprol, cilostazol, cinchophen, cinmetacin, cinnarizine, cipramadol, citalopram, clebopride, clemastine, clobenzepam, clocapramine, clomacran, clometacin, clometocillin, clomipramine, clonidine, clonitazene, clonixin, clopenthixol, clopriac, clospirazine, clothiapine, clovoxamine, cloxacillin, clozapine, codeine, cotinine, cromolyn sodium, cyamemazine, cyclacillin, cyclizine, cyclobenzaprine, cyclosporin A, cyproheptadine, deprenyl, desflurane, desipramine, dexamethasone sodium phosphate, dexfenfluramine, dexmedetomidine, dextroamphetamine, dextromoramide, dextropropoxyphene, diamorphine, diazepam, diclofenac, dicloxacillin, dihydrocodeine, dihydroergokryptine, dihydroergotamine, diltiazem, diphenhydramine, diphenicillin, diphenidol, diphenoxylate, dipipanone, disulfiram, dolasetronmethanesulfonate, domeridone, dornase alfa, dosulepin, doxepin, doxorubicin, doxylamine, dronabinol, droperidol, droprenilamin HCI, duloxetine, eletriptan, eliprodil, enalapril, enciprazine, enflurane, entacapone, entonox, ephedrine, epinephrine, eptastigmine, ergolinepramipexole, ergotamine, ergotamine tartrate, etamiphyllin, etaqualone, ethambutol, ethoheptazine, etodolac, famotidine, fenfluramine, fentanyl, fexofenadine, fientanyl, flesinoxan, fluconazole, flunisolide, fluoxetine, flupenthixol, fluphenazine, flupirtine, flurazepam, fluspirilene, fluticasone propionate, fluvoxamine, formoterol fumarate, frovatriptan, gabapentin, galanthamine, gepirone, ghrelin, glutathione, granisetron, haloperidol, halothane, heliox, heptylpenicillin, hetacillin, hydromorphone, hydroxyzine, hyoscine, ibuprofen, idazoxan, iloprost, imipramine, indoprofen, insulin (recombinant human), ipratropium bromide, iproniazid, ipsapiraone, isocarboxazid, isoetharine hydrochloride, isoflurane, isometheptene, isoniazid, rifampin, pyrazinamide, ethambutol, isoproterenol, isoproterenol hydrochloride, isoproterenol bitartrate, isosorbide dinitrate, ketamine, ketoprofen, ketorolac, ketotifen, kitanserin, lazabemide, leptin, lesopitron, levalbuterol hydrochloride, levodopa, levorphanol, lidocaine, lisinopril, lisuride, lofentanil, lofepramine, lomustine, loprazolam, loratidine, lorazepam, lorezepam, loxapine, maprotoline, mazindol, mazipredone, meclofenamate, mecloqualone, medetomidine, medifoxamine, melperone, memantine, menthol, meperidine, meperidine HCI, meptazinol, mesoridazine, metampicillin, metaproterenol, metaproterenol sulfate, methacholine chloride, methadone, methaqualone, methicillin, methprylon, methsuximide, methyphenidate, methyprylon, methysergide, metoclopramide, metofenazate, metomidate, metopimazine, metopon, metoprolol, metralindole, mianserin, midazolam, milnacipran, minaprine, mirtazapine, moclobemide; mofegiline, molindrone, mometasone furoate, morphine, nabilone, nadolol, nafcillin, nalbuphine, nalmefene, nalorphine, naloxone, naltrexone, naratriptan, nedocromil, sodium, nefazodone, nefopam, nicergoline, nicotine, nicotine, nifedipine, nisoxetine, nitrous oxide, nitroglycerin, nomifensine, nortriptyline, obestatin, olanzapine, omoconazole, ondansetron, orphenadrine, oxprenolol, oxycodone, palonosetron, papaveretum, papaverine, paroxetine, pemoline, penfluridol, penicillin N, penicillin O, penicillin S, penicillin V, pentamidine isethionate, pentazocine, pentetate, calcium trisodium, pentetate, zinc trisodium, pentobarbital, peptides, pergolike, pericyazine, perphenazine, pethidine, phenazocine, phenelzine, phenobarbital, phentermine, phentolamine, phenyhydrazine, phosphodiesterase-5, pilocarpine, pimozide, pipamerone, piperacetazine, pipotiazine, pirbuterol acetate, pirbuterolnaloxone, piroxicam, pirprofen, pizotifen, pizotyline, polyeptides, polypeptide YY, pramipexole, prentoxapylline, procaine, procaterol HCl, prochlorperazine, procyclidine, promazine, promethazine, propacetamol, propanolol, propentofylline, propofol, propoxyphene, propranolol, proteins, protriptyline, quetiapine, quinine, rasagiline, reboxetine, remacemide, remifentanil, remoxipride, retinol, ribavirin, rimonabant, risperidone, ritanserin, ritodrine, rizatriptan, roxindole, salicylate, salmeterol xinafoate, salmetrol, scopolamine, selegiline, sertindole, sertraline, sevoflurane, sibutramine, sildenafil, spheramine, spiperone, sufentanil, sulpiride, sumatriptan, tandospirone, terbutaline, terguride, testosterone, testosterone acetate, estosterone enanthate, testosterone proprionate, tetrahydrocannabinol, thioridazine, thiothixene, tiagabine, tianeptine, timolol, tiotropium bromide monohydrate, tizanidine, tobramycin, tofenacin, tolcapone, tolfenamate, tolfenamicacid, topiramate, tramadol, tranylcypromine, trazadone, triamcinolone acetonide, triethylperazine, trifluoperazine, trifluperidol, triflupromazine, trihexyphenidyl, trimeprazine, trimethobenzamide, trimipramine, tropisetron, tryptophan, valproicacid, vardenafil, venlafaxine, verapamil, vigabatrin, viloxazine, yohimbine, zafirlukast, zalospirone, zanamivir, zileuton, ziprasidone, zolmitriptan, zolpidem, zopiclone, zotepine, zuclopenthixol, and combinations thereof.

9. A device for administering a drug to the respiratory system of a patient, wherein the device delivers the drug to the patient using air supplied at a positive pressure relative to atmospheric pressure and wherein the patient is capable of unassisted breathing.

10. The device of claim 9, comprising a mask for delivering the drug to the patient, wherein the mask is substantially sealed around the patient's nose and mouth.

11. The device of claim 9, wherein the air is supplied at a pressure from about 1 cm H₂O to about 30 cm H₂O.

12. The device of claim 9, wherein the air is purified air.

13. A method of administering drugs to the respiratory system of a patient comprising:
delivering the drug to the patient using a gas supplied at a positive pressure relative to atmospheric pressure, wherein the patient is capable of unassisted breathing.

14. A device for administering a drug to the respiratory system of a patient, wherein the device delivers the drug to the patient using a gas supplied at a positive pressure relative to atmospheric pressure and wherein the patient is capable of unassisted breathing.
